Europäisches Patentamt

European Patent Office

Office européen des brevets

(11) Veröffentlichungsnummer: **0 600 298 A1**

# (12) EUROPÄISCHE PATENTANMELDUNG

(21) Anmeldenummer: **93118494.9**

(51) Int. Cl.5: **A61B 5/024**, A61B 5/0285

(22) Anmeldetag: **16.11.93**

(30) Priorität: **16.11.92 DE 4238641**
**08.07.93 DE 4322860**

(43) Veröffentlichungstag der Anmeldung:
**08.06.94 Patentblatt 94/23**

(84) Benannte Vertragsstaaten:
**AT BE CH DE DK ES FR GB IE IT LI NL PT SE**

(71) Anmelder: **LAUMANN MEDIZINTECHNIK GmbH**
**Längenauerstrasse 109**
**D-95100 Selb(DE)**

(72) Erfinder: **Bilz, Dietrich, Dr.**
**Mellenseestrasse 27**
**D-10319 Berlin(DE)**
Erfinder: **Laumann, Heiner**
**Silberbach 68**
**D-95100 Selb(DE)**
Erfinder: **Krauss, Manfred, Prof. Dr.**
**Am Bernsdorfer Hang 25**
**D-09126 Chemnitz(DE)**
Erfinder: **Waldmann; Jürgen, Prof. Dr.**
**Otto-Grothewohl-Strasse 13a**
**D-10117 Berlin(DE)**

(74) Vertreter: **Kruspig, Volkmar et al**
**Patentanwälte**
**Meissner, Bolte & Partner**
**Postfach 86 06 24**
**D-81633 München (DE)**

(54) **Arbeitsverfahren zum Betreiben und Vorrichtung zur Bestimmung und Auswertung des Zustandes von Gefässsystemen.**

(57) Die Erfindung betrifft ein Arbeitsverfahren zum Betreiben und eine Vorrichtung zur Bestimmung und Auswertung des Zustandes von Gefäßsystemen, insbesondere Blutgefäßen auf photoplethysmographischer Basis.

Mit dem Verfahren zur Bestimmung des Zustandes von Gefäßsystemen wird auf der Basis der Untersuchung von Mikro- und Makrozirkulationsvorgängen erreicht, daß eine erhöhte Informationsmenge aus dem zu untersuchenden System bereitsteht, wobei die Informationsmenge den Systemzuständen einfach zuordenbar ist, wodurch die Auswertung vereinfacht wird. Im erfindungsgemäßen Verfahren steht die Erfassung von zeitbezogenen und Kenngrößen im Vordergund, so daß auf die fehlerhafte Bestimmung und Berücksichtigung von Amplitudenwerten verzichtet werden kann. Mit dem Verfahren ist einerseits die Beschreibung des stationären Zustandes eines zu untersuchenden Gefäßsystems auf der Basis der Bestimmung der Mikrozirkulation und/oder andererseits die Ermittlung des dynamischen Verhaltens des zu untersuchenden Systems durch Anlegen einer definierten ein- oder mehrmaligen Sprungfunktion und Auswertung der Sprungübergangsfunktion bzw. -funktionen möglich. Zusätzlich kann durch die Anwendung des Doppler-Prinzips in Verbindung mit der Auswertung der Sprungübergangsfunktion der Ort einer möglichen Störung erkannt werden. Das optimale Zusammenwirken zwischen Herz, Blutkreislauf und Peripherie läßt sich durch eingeführte relative periphere Widerstände, die sich aus den Mikro- und Makrozirkulationsvorgängen im Echtzeitverfahren ableiten lassen, bestimmen.

Rank Xerox (UK) Business Services
(3.10/3.09/3.3.4)

EP 0 600 298 A1

Figur 29

Die Erfindung betrifft ein Arbeitsverfahren zum Betreiben und eine Vorrichtung zur Bestimmung und Auswertung des Zustandes von Gefäßsystemen gemäß dem Oberbegriff der Patentansprüche 1 bzw. 33.

Es ist bekannt, daß die Blutfülle der Mikrogefäße bestimmten rhythmischen Schwankungen unterliegt. In den letzten Jahren erfolgten umfangreiche Bemühungen, um die komplexen Ursachen dieser zeitlichen Veränderungen zu erkennen und aus diesen diagnostische Schlüsse zu ziehen.

Zur Erfassung der Makro- und Mikrozirkulations-Rhythmik werden auf der Basis optoelektronischer Komponenten, nämlich quasimonochromatischer Lichtquellen und entsprechender Photodetektoren, bestimmte zu untersuchende Hautareale optisch abgetastet, wobei nach erfolgter Gefäßentleerung auf der Basis der Auswertung von reflektiertem Licht auf eine Füllungszunahme in den cutanen Mikrogefäßen geschlossen wird.

Eine Einrichtung zur Erfassung und Analyse des Durchblutungszustandes der menschlichen Haut ist beispielsweise aus der deutschen Patentschrift DE 33 18 746 C2 bekannt geworden. Die Einrichtung greift auf das Prinzip der Licht-Reflexions-Rheographie (LRR) zurück. Hierbei wird eine Meßeinrichtung verwendet, welche mit einem leichten, an einem zu untersuchenden Hautareal mit doppelt klebenden Folienringen zu befestigenden Meßkopf ausgerüstet ist, wobei der Meßkopf Öffnungen aufweist, in denen ein optischer Strahlungsempfänger und mehrere Strahlungsquellen angeordnet sind und wobei der Strahlungsempfänger und die Strahlungsquellen unmittelbar an der Hautoberfläche liegen. Die Strahlungsquelle emittiert Strahlungen mit einer Wellenlänge im nahen IR-Bereich des Spektrums. Mit einer elektronischen Auswerteschaltung erfolgt ein selektives Erfassen und Aufzeichnen des zeitlichen Verlaufes des reflektierten bzw. zurückgestreuten Strahlungsanteils bei gleichzeitiger Unterdrückung der Volumenpulsationen. Die oben erwähnte Meßeinrichtung ist in der europäischen Patentschrift 0 063 649 B1 offenbart.

Es besteht jedoch ein wesentlicher Nachteil darin, daß die bekannte Auswertung der LRR-Kurven nur einige wenige diagnostisch relevante Aussagen ermöglicht. Durch die bisher ausschließlich subjektive Beurteilung der erhaltenen LRR-Kurven ist die Wahrscheinlichkeit einer Fehlinterpretation hoch.

Mit der bereits erwähnten deutschen Patentschrift 33 18 746 sollen die LRR-Signale objektiv und frei von subjektiven Entscheidungen analysiert und neue geeignetere Parameter zur fundierten sicheren Diagnose des Durchblutungszustandes der menschlichen Haut gewonnen werden.

Hierfür wird vorgeschlagen, das im allgemeinen erhaltene analoge Ausgangssignal in ein digitales Signal umzusetzen und selbiges einer sogenannten Rechenschaltung zuzuführen. Die Rechenschaltung soll dann physikalische Bewertungsparameter für die analogen LRR-Kurven berechnen. Hierfür wird mittels der Rechenschaltung das Ausgangssignal frequenzanalysiert und zusätzlich zur Bestimmung des Amplitudenverlaufes der Kreislaufrhythmik auch die Frequenzzusammensetzung selbiger ermittelt. In ausgewählten Frequenzbereichen erfolgt eine Fourier-Transformation des Ausgangssignales der Meßeinrichtung.

Als Bewertungsparameter wird die Auffüllzeit der Blutgefäße $t_0$, die Abfallzeit $t_a$ von einem Meßwert 90% auf einen Meßwert 10%, und die Halbwertzeit $t_h$, d. h. die Zeit, in der ein Abfall von 100% auf 50% erfolgt, bestimmt.

Die Computeranalyse der LRR-Kurven wird bei der bekannten Lösung in vier Punkten zusammengefaßt. Es werden zunächst die Ergebnisse der Berechnung der venösen Auffüllphase ausgegeben. Neben der Bestimmung der Auffüllzeit $t_0$ werden die von den störanfälligen Randbedingungen befreite Abfallzeit $t_a$, die bereits erwähnte Halbwertzeit und weitere Parameter berechnet. Danach erfolgt anhand der Amplitudenwerte der gespeicherten LRR-Kurve die Berechnung der Druckdifferenz durch das Auffüllen der Gefäße und bei Belastung. Als Bewertungsparameter wird weiterhin die Fläche unter der LRR-Kurve oder die Steilheit der Auffüllphase zur Diagnose des Zustandes der venösen Hämodynamik herangezogen.

Durch die Möglichkeit des Transfers der Meßergebnisse aus dem Zeit- in den Frequenzbereich und Ausgabe eines Amplitudendichte-Spektrums mit der Angabe bestimmter Amplitudenfaktoren des Fourier-Spektrums sollen weitere Ansatzpunkte zur Verbesserung der Diagnostik gegeben sein.

Mit der vorstehend skizzierten Lösung wird es zwar möglich, die erhaltene Informationsmenge aus der LRR-Untersuchung zu vergrößern und eine Vielzahl von Einzelmeßergebnissen bereitzustellen, jedoch wird durch das Prinzip der rechnergestützten Mustererkennung oder den Datentransfer in die Frequenzdomäne die Auswertung für den behandelnden Arzt, der in der Lage sein muß, schnell und mit hoher Treffsicherheit in der täglichen Praxis zu diagnostizieren, nicht erleichtert.

Die zugrundegelegten Auswerteparameter sind für eine gesicherte Diagnose nicht ausreichend.

Die Aufgabe der Erfindung besteht daher darin, ein Arbeitsverfahren zum Betreiben und eine Vorrichtung zur Bestimmung und Auswertung des Zustandes von Makro- und Mikrogefäßsystemen auf der Basis einer qualitativ neuen Photoplethysmographie anzugeben, wobei der klinische Informationsgehalt der erfaßten Biosignale wesentlich erhöht und eine umfassende relevante Zuordnung von statistisch gesicherten Merkmalsänderungen zu entsprechenden pathophysischen und klinischen Zuständen möglich werden.

Die Lösung der erfindungsgemäßen Aufgabenstellung erfolgt mit den Merkmalen der Patentansprüche 1, 18, 19, 23, 25, 33, 35, 36 und 39, wobei die Unteransprüche zweckmäßige Ausgestaltungen und Weiterbildungen der Erfindung zeigen.

Die Erfindung geht dabei von dem Grundgedanken aus, daß es möglich ist, das dynamische Verhalten eines linearen Systems dadurch zu ermitteln, daß das zu untersuchende System mit einer definierten Eingangsfunktion $x_e(t)$, wobei diese zum Beispiel eine Sprung- oder Deltafunktion sein kann, beaufschlagt wird. Als Ergebnis oder Wirkung $x_a(t)$ erhält man am Systemausgang eine typische Sprung- bzw. Stoßübergangsfunktion. Diese Funktion charakterisiert das dynamische Systemverhalten. Zweckmäßigerweise werden hierbei im allgemeinen dimensionslose bzw. normierte Größen angestrebt. Das dynamische Verhalten der Sprungantwort bzw. Ausgangsgroße $x_a(t)$ läßt sich durch Kennwerte, die man aus dem Funktionsverlauf von $x_a(t)$ ableiten kann, beschreiben. Typisch ist zum Beispiel die Einschwingzeit $t_E$. Es wurde erkannt, daß solche zu beschreibenden Signale und Systeme näherungsweise auch im Fall der Biomedizin, d. h. bei der Makro- und Mikrozirkulation in Blutgefäßen, vorliegen. Es wurde gefunden, daß das dynamische Verhalten photoplethysmographisch gewonnener Volumenpulse ein Ausdruck derartiger Funktionen ist. Vorteilhafterweise wird ein negativer und anschließend ein positiver Sprung erzeugt, wobei die Sprünge zu einer Entleerung bzw. Füllen des Gefäßsystems führen.

Erfindungsgemäß wird eine Sprungfunktion durch plötzliche, einmalige Einwirkung, zum Beispiel des plötzlichen Hebens einer Hand oder eines Beines, eines akustischen Signals oder in Form einer druckmechanischen Belastung, ausgelöst. Die Ausgangsgröße des Systems, d. h. des zu untersuchenden peripheren Gebietes, zum Beispiel einer Großzehe, stellt sich dann entsprechend dem jeweiligen konkreten Zustand ein.

Es wurde weiterhin erkannt, daß sich bei jedem zu untersuchenden System eine Aussteuerung in bzw. um einen Arbeitspunkt AP ergibt. Dieser Arbeitspunkt AP liegt auf der Systemkennlinie, die sich als Darstellung von $x_a$ über $x_e$ ergibt.

Hierbei existieren normale bzw. optimale Werte. Von diesen normalen bzw. optimalen Werten wird im Krankheitsfalle abgewichen. Durch einen einfachen Vergleich der normalen bzw. optimalen Werte mit den jeweils aktuell ermittelten Werten läßt sich mit großer Sicherheit der tatsächliche Zustand des untersuchten peripheren Gebietes feststellen.

Es wurde gefunden, daß sich bei der Mikrozirkulation im Sinne der Chaos-Theorie eine Grundordnung, d. h. ein Arbeitspunkt, einstellt. Daneben besteht determiniertes Chaos. Um den Arbeitspunkt zu bestimmen, wird im stationären Zustand des zu untersuchenden Systems über eine bestimmte Meßzeit die Mikrozirkulation ermittelt.

Bereits hieraus läßt sich erfindungsgemäß schlußfolgen, daß zum Beispiel aus der Analyse der Streubreite der Herzfrequenz erkannt werden kann, ob ein Nichtnormalzustand vorliegt. Es wurde erkannt, daß je kleiner die Streubreite der Herzfrequenz, je größer die Gefährdung oder die potentielle Wahrscheinlichkeit einer Erkrankung ist.

Durch die vorstehend beschriebene Verfahrensweise lassen sich dann unmittelbar und ohne subjektive Einflußnahme des Probanden oder eines Dritten beispielsweise Auswirkungen von Pharmaka oder Drogen feststellen.

Es liegt im Sinne der Erfindung, daß das Arbeitsverfahren zum Betreiben einer Vorrichtung zur Bestimmung und Auswertung des Zustandes von Gefäßsystemen bei turnusmäßigen Flugtauglichkeitsuntersuchungen oder ähnlichen Tauglichkeitsuntersuchungen angewendet werden kann. Darüber hinaus besteht die Möglichkeit, durch eine periodische oder kontinuierliche Anbordüberwachung z. B. eines Flugzeugführers oder im Weltraumeinsatz die Leistungsfähigkeit des Gefäßsystems und damit den physischen Zustand des Flugzeugführers bzw. des Probanden zu bewerten. Diese Bewertung erfolgt objektiv. Aus der Bewertung können bereits vor dem subjektiven Empfinden eingeschränkter Leistungsfähigkeit Maßnahmen zur Vermeidung von Havarien und von Fehlbedienungen aufgrund eingeschränkter Reaktionsfähigkeit eingeleitet werden.

So ist z.B. der Beginn des Herztodes gekennzeichnet durch den Übergang von einem irregulären Muster der Herztätigkeit zu einem einfachen Muster.

Aus der Analyse der Mikrozirkulation in einem periphären Gebiet (Geweberegion) ist es daher möglich, durch einfache Überwachung der Kontinuität oder Diskontinuität der Herztätigkeit bzw. der Herzfrequenz eine Herzerkrankung festzustellen oder einen bevorstehenden Herzstillstand rechtzeitig zu erkennen.

Erfindungsgemäß läßt sich die photoplethysmographisch ermittelte integrale Gesamt-Volumenpulsation für das jeweilige ausgeleuchtete periphere Gebiet wie folgt beschreiben:

4

$$x_{ges}(t) = x_I(t) + x_{II}(t) + x_{III}(t) + \ldots + x_N(t)$$
$$= x_{mikro}(t)$$

Dabei bedeuten:

$x_{ges}(t)$, $x_{mikro}(t)$:      integrale Gesamt-Volumenpulsation des photoplethysmographisch untersuchten peripheren Gebietes (Mikrozirkulationsfunktion)

$x_I(t)$:      Welle I. Ordnung, verbunden mit der Herzfrequenz als Trägerfrequenz der Mikrozirkulation

$x_{II}(t)$:      Welle II. Ordnung, verbunden mit der Atmung

$x_{III}(t)$:      Welle III. Ordnung, verbunden mit der Blutdruckperiode (ca 10 s - Rhythmus)

$x_N(t)$:      Wellen N-ter Ordnung (bis hin zu mehrtägigen Schwankungen).

Es wurde erkannt, daß alle oben erwähnten Teil-Funktionen den Gesetzen des determinierten Chaos unterliegen.

Das erfindungsgemäße Verfahren auf der Basis der Auswertung von Mikro- und Makrozirkulationsvorgängen in Blutgefäßen geht aufgrund der oben geschilderten Erkenntnisse von der stochastischen Funktion $x_{ges}(t)$ im Zeitbereich aus.

Das bereits erwähnte Maß $x_I(t)$ für die Wellen I. Ordnung enthält die "Trägerfrequenz" der Mikrozirkulationsfunktion, die Herzfrequenz (Herzperiodendauer $T_I$). Um einen solchen Arbeitspunkt $T_I$ wird ausgesteuert, wobei die Streuung $S_T$ auftritt. Ein einfacheres Herzrhythmusmuster bedeutet, daß die Streuung $S_T$ immer mehr abnimmt und der Arbeitspunkt sich außerhalb des optimalen Bereiches befindet. Im Sinne des erfindungsgemäßen Verfahrens sind daher der Arbeitspunkt und die Streuung für den sogenannten Normalfall in Abhängigkeit vom jeweiligen Lebensalter sowie das mögliche Optimum zu ermitteln und für die weitere Auswertung als Vergleichswerte oder in Form einer Normalkennlinie abzuspeichern.

In einer Ausführungsform der Erfindung läßt sich die Funktion $X_I(t)$ aus $x_{ges}(t)$ durch einen Bandpaß mit einer Grenzfrequenz 0,6 Hz < f < 9 Hz ausblenden, trennen und darstellen.

Das Maß für die Wellen höherer Ordnung läßt sich mit $x_{bio}(t)$ zusammenfassen.

Diese Wellen höherer Ordnung, die langwellig sind, lassen sich ebenfalls aus $x_{ges}(t)$ durch einen Tiefpaß bzw. Bandpaß mit einer oberen Grenzfrequenz $f_g$ von ungefähr 0,5 ... 0,6 Hz ausfiltern.

Aus dem konkreten Verlauf $x_I(t)$ sind nun eine Vielzahl von Parametern, einschließlich der erwähnten Streuungen S, ermittelbar.

So wird eine Gipfelamplitude A, eine Dikrotieamplitude B, eine Gipfelzeit $T_G$, eine Dikrotiezeit $T_D$, eine arterielle Grundschwingung $T_{AG}$ und eine Pulsperiodenzeit (Herzperiodendauer) $T_I$ von $x_I(t)$ ermittelt.

Erfindungsgemäß wurde erkannt, daß ein optimaler Arbeitspunkt für den gesunden Menschen bei folgenden Verhältnissen vorliegt:

$T_D/T_I = 0,5$

$T_G/T_I = 0,191$

$T_{AG}/T_I = 0,309$ sowie

$T_{AG}/T_D = 0,618$

$T_G/T_D = 0,382$.

Das ideale Amplitudenverhältnis AV im Arbeitspunkt ergibt sich nach der Beziehung:

$AV_{ideal} = B/A = 0,618$.

Mit den erwähnten Beziehungen läßt sich im Sinne der Vereinfachung des Auswerteverfahrens ein Optimum in Form eines Merkmalvektors definieren.

Da Amplitudenwerte der photoplethysmographisch ermittelten Mikrozirkulation von verschiedenen Faktoren abhängig sind, wird erfindungsgemäß die Gleichwertigkeit der Zeitkennwerte bei der Volumenpulsation erkannt. Damit wird eine qualitativ und quantitativ neue Photoplethsymographie, eine zeitdiskrete Photoplethsymographie, geschaffen und der klinische Informationsgehalt beträchtlich erhöht, da die Zeitkennwerte $T_I$, $T_D$, $T_G$, $T_{AG}$ weitgehend unabhängig von Andruck sind und eine Eichung entfällt. Aus Gründen der Vergleichbarkeit wird auf eine Normierung (z. B. auf Herzperiodendauer $T_I$) orientiert.

Es besteht nun ein weiterer Grundgedanke der Erfindung darin, bestimmte Merkmalsvektoren bestimmten Krankheitsbildern zuzuordnen. Hierfür werden erfindungsgemäße Bewertungskriterien aufgestellt, die eine optimale Entscheidungsfindung ermöglichen.

Zur Erhöhung der Sicherheit gegenüber Störungen bei der Ermittlung der Mikrozirkulationsfunktionen werden erfindungsgemäß fehlererkennende und fehlerkorrigierende Codes angewendet. Grundsätzlich wird hierbei eine zusätzliche Redundanz in den jeweiligen Code eingebaut.

Ein fehlerkorrigierendes Verfahren besteht darin, diese Redundanz dadurch zu realisieren, daß die jeweiligen Bewertungen wiederholt und deren Ergebnisse verglichen werden. Bei Übereinstimmung ist kein Fehler vorhanden, bei Nichtübereinstimmung wird eine nochmalige Wiederholung durchgeführt, wobei mit großer Wahrscheinlichkeit die zwei übereinstimmenden Codewörter (Kennwerte) als richtiges Codewort erkannt und damit eine Fehlerkorrektur ermöglicht werden kann.

In einer weiteren Ausführungsform der Erfindung wird erkannt, daß sich das komplexe System des Blutkreislaufs im Sinne eines Modells auf den Grundstromkreis der Elektrotechnik zurückführen läßt und somit das Zusammenwirken des Herzens mit dem gesamten Blutkreislaufsystem einschließlich Peripherie durch eine Spannungsquelle E, einem zugehörigen Innenwiderstand $R_i$ und einen Verbraucher (Belastungswiderstand) $R_a$ modellierbar ist. Dabei wird das Maximum der vom Herzen in die Peripherie (Fuktionseinheit Mikrozirkulation) übertragenen Leistung dann erreicht, wenn der Innenwiderstand $R_i$ und der Belastungswiderstand $R_a$ gleich groß sind, d. h.

$R_i = R_a$ ist.

Dieses Optimum wird als Anpassung bezeichnet. Ist $R_i > R_a$, liegt eine Unteranpassung, bei $R_i < R_a$ eine Überanpassung vor, so daß eine entsprechend geringere Leistung übertragen wird. Aus diesem Modell werden erfindungsgemäß als Innenwiderstand $R_i$ der "relative periphere Widerstand" $RPW_1$ eingeführt, der näherungsweise das Verhältnis von systolischem zu diastolischem Blutdruck $P_S$ und $P_D$ darstellt:

$\overline{RPW_1} \approx P_S/P_D.$

Es wurde erfindungsgemäß des weiteren erkannt, daß dieser dimensionslose Widerstand einen optimalen Wert (Norm-Wert) besitzt

$\overline{RPW_1} = \overline{RPW_1}\ _{opt} = 1{,}618$

und $\overline{RPW_1}$ als Kennlinie darstellbar ist, die ausgesteuert wird, so daß Aussteuerungsgrenzen angebbar sind (z.B. Normotonie, Grenzwerthypertonie). Da die Blutdruckamplitude $\Delta P$ die Differenz zwischen systolischem und diastolischem Blutdruck darstellt, läßt sich erfindungsgemäß aufgrund der Gültigkeit des Prinzips der Selbstähnlichkeit im Mikro- und Makrobereich und Anwendung der Strahlensätze setzen:

$\Delta P/P_D = T_B/T_A,$

wobei $T_B$ die systolische Gipfelzeit im Blutdruckverlauf, $T_A$ die Abfallzeit zwischen dem systolischen Gipfel und der dikroten Einkerbung (Inzisur) darstellen. Somit können erfindungsgemäß im Echtzeitverfahren für jede Herzperiode durch Messung der Zeitkennwerte $T_B$, $T_A$ z. B. mittels Ultraschalldopplerverfahren die relativen Blutdruckwerte sowie der relative periphere Widerstand $\overline{RPW_1}$ ermittelt werden. $\overline{RPW_1}$ stellt dabei den integralen Mittelwert dar. Es gilt folglich für die n-te Herzperiode der Zusammenhang:

$RPW_1(n) = 1 + T_B(n)/T_A(n) \triangleq P_S(n)/P_D(n),$

woraus sich der Mittelwert ergibt:

$$\overline{RPW_1} = 1/N \sum_{n=1}^{n} RPW_1(n).$$

Erfindungsgemäß wurde aus dem zugrundeliegenden Modell erkannt, daß als ein "relativer peripherer Widerstand" $RPW_2$ aus jedem Volumenpuls n abgeleitet werden kann:

$RPW_2(n) = 1 + T_G(n)/T_{AG}(n) = T_D(n)/T_{AG}(n),$

6

wenn $T_G$ die Gipfelzeit, $T_D$ die Dikrotiezeit und $T_{AG}$ die arterielle Grundschwingung einer Volumenpulsperiode darstellen. Als Mittelwert $\overline{RPW_2}$ gilt analog zu $\overline{RPW_1}$

$$\overline{RPW_2} = 1/N \sum_{n=1}^{N} RPW_2(n) \approx 1 + T_G/T_{AG} = T_D/T_{AG}.$$

Überraschenderweise erhält man als Optimum bzw. Norm-Wert analog zu $RPW_1$

$\overline{RPW_2} = \overline{RPW_2}$ opt = 1,618.

Erfindungsgemäß wird nun erkannt, daß der im Blutkreislauf (Grundstromkreis) sich einstellende relative mittlere (Blut-) Stromfluß nach der allgemeinen Beziehung

$I_{rel} = 3,236 / \overline{RPW_1} + \overline{RPW_2}$

verläuft, wobei $I_{rel\ opt} = 1$ mit $\overline{RPW_1} = \overline{RPW_2} = 1,618$ beträgt. Es wurde des weiteren erkannt, daß im Sinne des zugrundegelegten Modells das Zusammenwirken des Herzens mit der Peripherie über das Blutkreislaufsystem durch einen Algorithmus beschreibbar ist, wobei sich im optimalen Fall

$\overline{RPW_1} \approx \overline{RPW_2} \approx \overline{RPW_{opt}} = 1,618$

einstellt. Dann gilt $R_i = R_a$.

Als Sollwert für die Herzperiodendauer $T_H$ wurde erfindungsgemäß dabei der Zusammenhang erkannt, daß

$T_{H\ SOLL}$ [ms] $\approx 508 [1 + 1/\overline{RPW_1}] \approx 508 [1 + P_D/ P_S]$ ist,

wobei die Differenz $[T_{H\ IST} - T_{H\ SOLL}]$ unter Beachtung der Aussteuerungsschranken für $P_S$ und $P_D$ für eine einzuleitende Therapie (Veränderung des Herzminutenvolumens als Produkt von Schlagvolumen und Herzfrequenz) zugrunde zu legen ist.

In einer weiteren Ausführungsform der Erfindung beruht das Verfahren zur Auswertung von Mikro- und Makrozirkulationsvorgängen in Blutgefäßen auf einer Darstellung der aus $x_{ges}(t)$ abgeleiteten verallgemeinerten Mittelwertfunktion: der Autokorrelationsfunktion $\psi_{xges}(\tau)$. Zusätzlich wird ein sogenannter Mikrozirkulationsgradient $M_{mikro}$ und eine Kreuzkorrelationsfunktion $\psi_{xy}(\tau)$ für zwei Mikrozirkulationssignale eingeführt.

Es wird zunächst analysiert, wie in $x_{ges}(t)$ die Wellen verschiedener Ordnung $x_I(t)$, $x_{II}(t)$, $x_{III}(t)$ anteilig im statistischen Mittel enthalten sind und welche mittleren Perioden auftreten.

Hieraus wird erfindungsgemäß jeweils eine Autokorrelationsfunktion (AKF) (für die Teilfunktionen $x_I(t)$, $x_{II}(t)$, $x_{III}(t)$) gebildet unter der Annahme, daß die Wellen N-ter Ordnung statistisch voneinander unabhängig sind. Es ergibt sich hieraus die Beziehung:

$\psi_{xges}(\tau) = \psi_{xI}(\tau) + \psi_{xII}(\tau) + \psi_{xIII}(\tau)$ ,

wobei $\psi_{xges}(\tau)$ die Autokorrelationsfunktion der integralen Gesamtvolumenpulsation darstellt. Allgemein liegt erfindungsgemäß die mittlere Periode von $\psi_{xI}(\tau)$ im Bereich 0,7 ... 1,3 s, die von $\psi_{xII}(\tau)$ im Intervall 3 ... 5 s, und von $\psi_{xIII}(\tau)$ im Bereich von ca. 10 ... 15 s.

Bei zeitlich entsprechend langen Meßsignalen für $x_{ges}(t)$ kann von einem stationären stochastischen Signal ausgegangen werden. Bei einer Signalwiederholung wird sich daher eine gleiche, typische Autokorrelationsfunktion $\psi_{xges}(\tau)$ ergeben.

Ebenso läßt sich darstellen:

$\psi_{xges}(\tau) = \psi_{xI}(\tau) + \psi_{xbio}(\tau)$

mit $\psi_{xbio}(\tau)$ als Autokorrelationsfunktion der Bio-Periodik.

Zur Auswertung wird nunmehr in einfacher Weise die Einhüllende von $\psi_{xges}(\tau)$ betrachtet, welche bei Vorhandensein von $x_{II}(t)$ und $x_{III}(t)$ außer in den Maxima und Minima keine Waagerechte bildet, sondern eine entsprechende tangentiale Neigung aufweist. Tritt eine Waagerechte als Tangente auf, ist der Anteil von $x_{bio}(t)$ erfindungsgemäß im statistischen Mittel Null. Erfindungsgemäß wird nunmehr der Mikrozirkulations-gradient $M_{mikro}$, welcher aus der Autokorrelationsfunktion $\psi x_{ges}(\tau)$ abgeleitet ist, definiert und zur Auswertung herangezogen.

$M_{mikro}$ ergibt sich dabei wie folgt:

$M_{mikro}$ = 1 - Min/Max mit

Max = Maximum der (nahezu) periodischen Funktion $\psi x_{ges}(\tau)$ und Min = zugehöriges Minimum.

Hieraus folgt, daß bei fehlender Zeitfunktion $x_{bio}(t)$ die Teil-AKF $\psi_{xbio}(\tau)$ = 0 und somit auch $M_{mikro}$ = 0 ist.

Die konkreten Werte im betrachteten bzw. zu untersuchenden System $M_{mikro}$ können in einfacher Weise ermittelt und mit statistisch gesicherten, beispielsweise in einer Tabelle gespeicherten Werten bestimmter abormaler Zustände verglichen werden.

Analog zur Autokorrelationsfunktion kann auch die Kreuzkorrelationsfunktion als Maß zur Bewertung herangezogen werden. Wird die Kreuzkorrelationsfunktion von den Signalen $x_1(t)$ und $x_2(t)$ zum Beispiel am gleichen Finger oder Zehe, jedoch an den verschiedenen Meßstellen 1 und 2 ermittelt, und wird das Maximum bei $\tau$ = 0 erhalten, so sind die integralen Volumenpulsationen gleich.

Aus der Bestimmung der zeitlichen Lage $\tau_{opt}$ des Maximums von $\psi_{x1x2}(\tau)$ läßt sich die mittlere Blutströmungsgeschwindigkeit zwischen zwei Meßstellen in einfacher Weise ableiten.

Wie bereits eingangs erwähnt, werden erfindungsgemäß zur Ermittlung und Beurteilung des dynamischen Verhaltens des zu untersuchenden Gefäßgebietes definierte Eingangsfunktionen angelegt. Dies kann zum Beispiel durch eine sprung- oder stoßförmig veränderte Andruckkraft eines Sensors zur Erfassung des reflektierten Lichtes im untersuchten Gebiet erfolgen.

Die mit dem Sensor aufgenommene Antwortfunktion bzw. Sprungübergangsfunktion ist nunmehr Ausdruck für das dynamische Verhalten des von der Strahlung durchdrungenen Mikrozirkulations-Gebietes, d.h. der integralen Volumenpulsation der Mikrozirkulation.

Als Eingangsfunktion ist ein positiver oder aber auch ein negativer hydrostatischer Sprung denkbar. Die erhaltene Sprungantwort läßt sich nunmehr in einfacher Weise zur Auswertung heranziehen, indem z.B. nach der Größe der Einschwingzeit $t_E$ eine Klassifizierung durchgeführt wird. Damit wird auch bei dieser dynamischen Systemdiagnostik die zeitdiskrete Photoplethsymographie zugrunde gelegt.

Durch das Auslösen eines negativen und positiven hydrostatischen Drucksprungs entfällt das ansonsten erforderliche bekannte, komplizierte Bewegungsprogramm für Probanden bzw. Patienten. Es wird jedoch das Entleeren und daraus eine "Einschwing"- oder "Entleerzeit" $t_{EL}$ des Mikro- und verbundenen Makrogefäßsystems gemessen sowie als Folge des positiven hydrostatischen Drucksprunges das Wiederauffüllen mit der "Einschwing"- oder "Auffüllzeit $t_{EF}$ bestimmt. Es wurde erkannt, daß im Normalfall der Zusammenhang zwischen Entleeren und Füllen gilt

$t_{EF} \approx 1,6\ t_{EL}$,

bei Venenklappen-Insuffizienz jedoch die Einschwingzeiten gleich groß sind.

Mit einer weiteren Ausführungsform der Erfindung kann die Vasomotorik bestimmt werden.

Die Güte der Vasomotorik wird z. B. durch Auslösen eines akustischen Reizes (Stoß) als Eingangsgröße beurteilt. Die erhaltene Antwort $x_a(t)$ stellt sich als Stoßübergangsfunktion ein. Die Güte der Vasomotorik wird dann in einfacher Weise daraus ermittelt, indem die Einschwingzeit $t_E$ aus der Einhüllenden von $x_a(t)$ bestimmt wird. Bei fehlender Vasomotorik erhält man $t_E$ = 0. Als Einschwingzeit $t_E$ wird die Zeit verstanden, nach der die Antwort auf die Eingangs-Stoßfunktion innerhalb des Bereiches von ± 5% von $x_a(\infty)$ verbleibt.

Durch eine Auswertung von Zeitkennwerten der Volumenpulsation wird der Informationsgehalt beträchtlich erhöht. In diesem Sinne wurde erfindungsgemäß ein Vasomotorikgradient $M_{vaso\ 1}$ eingeführt. Als andere mögliche Kenngröße läßt sich aus den Amplitudenverhältnissen der Vasomotorikgradient $M_{vaso\ 2}$ einführen.

In einfacher Weise ist eine Klassifizierung von $M_{vaso}$ inverschiedene Vasomotorik-Stufen möglich.

Die Erfindung soll nun anhand mehrerer Ausführungsbeispiele und von Figuren näher erläutert werden. Hierbei zeigen:

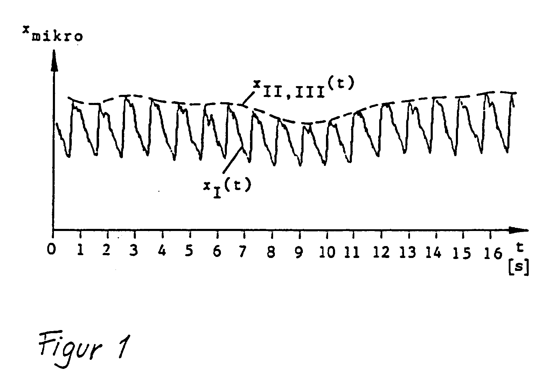

Fig. 1    den typischen Verlauf eines photoplethysmographisch ermittelten Mikrozirkulationssignals, bestehend aus den Komponenten $x_I(t)$, $X_{II,III}(t)$,

| | |
|---|---|
| Fig. 2 | einen Zeitausschnitt einer ermittelten Volumenpulsation mit charakteristischen Parametern, |
| Fig. 3 a-c | in Abhängigkeit vom Lebensalter die qualitativen Normalwerte von normierten Parametern der Volumen pulsation sowie die Verhältnisse im Optimalfall OPT, |
| Fig. 4 | den Mikrogefäßzustand als Darstellung der normierten Gipfelzeiten in Abhängigkeit von der normierten Dikrotiezeit und der zugehörigen Klassifizierung sowie den Übergangsbereich UE, |
| Fig. 5 | den Blutkreislauf als Grundstromkreis mit Spannungsquelle E (Herz), Innenwiderstand $R_i$ und Verbraucher $R_a$ (Funktionseinheit Mikrozirkulation), |
| Fig. 6 | normale und nichtnormale Formen des zentralen Pulses mit den Zeitkennwerten $T_A$, $T_B$ <br> A... normal <br> B, C, D ... nichtnormal, |
| Fig. 7 | die Abhängigkeit zwischen systolischem Blutdruck $P_S$ und $P_D$ mit verschiedenen Werten des relativen peripheren Widerstandes $RPW_1$ als Parameter, Ruheblutdruckwerten nach E. Stein und Aussteuerungsgrenzen, |
| Fig. 8 | eine mit einem Ultraschalldoppler aufgenommene Fließgeschwindigkeit in einer Armarterie einschließlich der charakteristischen Zeitparameter, |
| Fig. 9 | den ermittelten relativen peripheren Widerstand $RPW_2$, Dikrotiezeit $T_D$ und arterielle Grundschwingung $T_{AG}$ einschließlich Streubereich bei Zeitparametern in Abhängigkeit des Lebensalters, |
| Fig. 10 | den ermittelten peripheren Widerstand $RPW_2$, Herzperiodendauer $T_H = T_I$, Dikrotiezeit $T_D$ und arterielle Grundschwingung $T_{AG}$ je Volumenpuls einer aufgenommenen Mikrozirkulationsfunktion für drei charakteristische Probanden, |
| Fig. 11 | die normierte Verbraucherleistung $P_a{}^*$ in Abhängigkeit der relativen peripheren Widerstände ($P_A$ ... Verbraucherleistung, $P_K$ ... Leistungsabgabe des Generators bei Kurzschluß), |
| Fig. 12 a bis d | Normalbereiche in Abhängigkeit vom Lebensalter für den Ruheblutdruck (Fig. 12a), daraus abgeleitet $RPW_1$ (Fig. 12b), für $RPW_2$ (Fig. 12c) und für den relativen mittleren (Blut-) Stromfluß $I_{rel}$ (Fig. 12d). A, B, D = Meßwerte für Probanden nach Fig. 10. |
| Fig. 13 a und b | eine ermittelte Autokorrelationsfunktion $\psi_{xmikro}(\tau)$ mit (a) entsprechenden abgeleiteten Kenngrößen und (b) mit stark ausgeprägter Atmungskomponente, |
| Fig. 14 | eine prinzipielle Darstellung zur Ermittlung der mittleren Blutströmungsgeschwindigkeit, |
| Fig. 15 | die Definition der Einschwingzeit $t_E$, |
| Fig. 16 | die Messung der Einschwingzeit zur Beurteilung der Vasomotorik bei einer durch akustische Sympathikusstimulation hervorgerufenen Änderung der Mikrozirkulation (Anzahl der durch die Vasomotorik erfaßten Volumenpulsationen: n = 16; Diagnose: Klasse I/gesund), |
| Fig. 17 | prinzipielle Darstellungen zur Ermittlung des Vasomotorikgradienten, |
| Fig. 18 | einen Systemtest mit negativem und positivem hydrostatischem Drucksprung, |
| Fig. 19 | eine hämodynamische Gradeinteilung für das Entleeren und Füllen der Mikro- und Makrogefäße bei einem Systemtest nach Fig. 18, |
| Fig. 20 | eine Darstellung der Systemeingangsgröße hydrostatischer Drucksprung, |
| Fig. 21 | praktische Ergebnisse eines Systemtestes "venöses Entleeren und Füllen", |
| Fig. 22 | ein Vasolineal, |
| Fig. 23 | simultan aufgenommene Mikrozirkulationsfunktionen bei einem Diabetiker im fortgeschrittenen Zustand <br> a) am rechten Zeigefinger, <br> b) an der rechten Großzehe, |
| Fig. 24 | aus den Verläufen nach Fig. 23 ermittelte Autokorrelationsfunktionen <br> a) für rechten Zeigefinger, <br> b) für rechte Großzehe, |
| Fig. 25 | Darstellung der Funktionseinheit Mikrozirkulation, |
| Fig. 26 a und b | den Verlauf des arteriellen Zuflusses, die gespei  cherte Mengenveränderung pro Zeiteinheit sowie den venösen Abfluß bei der Funktionseinheit Mikrozirkulation, falls eingangsseitig ein negativer und positiver hydrostatischer Drucksprung angelegt wird, |

a) den qualitativen Verlauf mit entsprechenden Kenngrößen (Pulsationen in der Mikrozirkulation sind nicht dargestellt), insbesondere mit den Gleichgewichtspunkten GP 0, 1, 2 sowie $t_{EL\ venös}$,

b) den bei einem Probanden gemessenen quantitativen Verlauf mit einem Multiplex-Rot/NIR-Dopplersensor,

Fig. 27          den prinzipiellen Aufbau eines dynamischen Ultraschall-Dopplers;

Fig. 28 a und b     eine mit einem dynamischen Ultraschall-Doppler aufgenommene venöse (a) und arterielle (b) Sprungübergangsfunktion.;

Fig. 29          ein Blockschaltbild einer Realisierungsform der Vorrichtung zur Gefäßzustandsbestimmung.

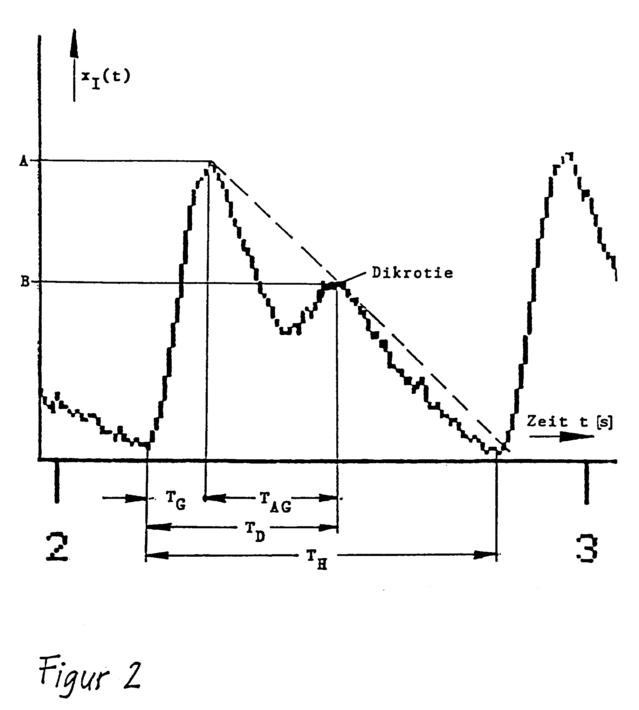

Mit Hilfe der Fig. 1 und 2 soll in einem Ausführungsbeispiel illustriert werden, wie das erfindungsgemäße Verfahren eine Auswertung der stationären, stochastischen Funktion $x_{ges}$ (t) im Zeitbereich ermöglicht.

Die Fig. 2 zeigt einen Zeitausschnitt einer ermittelten Volumenpulsation nebst zugehörenden Parametern. Hierbei bedeuten:

A          Gipfelamplitude

B          Dikrotieamplitude

$T_G$          Gipfelzeit

$T_D$          Dikrotiezeit

$T_{AG}$          arterielle Grundschwingung und

$T_I = T_H$          Pulsperiodenzeit von $x_I(t)$ (Herzperiodendauer).

Alle Parameter besitzen im Sinne der Chaostheorie eine Streuung S, z.B. $S_{TI}$ als Streuung von $T_I$.

Aus der Fig. 2 geht hervor, daß die entsprechenden Informationen sowohl in der Amplitude als auch in der Zeit enthalten sind.

Die Amplituden des photoplethysmographisch ermittelten Signals haben den Nachteil, daß sie andruckabhängig sind, z.T. von multiplen von außen wenig zu beeinflussenden Faktoren bestimmt werden und kaum eichbar sind. Mit der gleichwertigen Auswertung von Zeitkennwerten wird ein ähnlicher Qualitätssprung wie in der Nachrichtentechnik beim Übergang von der Amplitudenzur Frequenzmodulation erreicht. Um die Ergebnisse verallgemeinern zu können, wird ebenfalls auf relative (bezogene bzw. normierte) Größen orientiert. Zentrale Parameter einer Normierung sind die Herzperiodendauer $T_I = T_H$ sowie für die Ermittlung des relativen peripheren Widerstandes $RPW_2$ die arterielle Grundschwingung $T_{AG}$.

Aus der Analyse einer großen Anzahl von Meßsignalen und der prinzipiellen Darstellung des Verlaufes nach Fig. 2 werden in überraschender Weise als optimale Verhältnisse gefunden $T_D/T_I = 0{,}5$, $T_G/T_I = 0{,}191$ $T_{AG}/T_I = 0{,}309$; jedoch ebenso $B/A = 0{,}618$. Diese Werte stellen ideale Arbeitspunkte dar.

Als allgemeine Grundbeziehung läßt sich des weiteren angeben

$$T_D = T_G + T_{AG}.$$

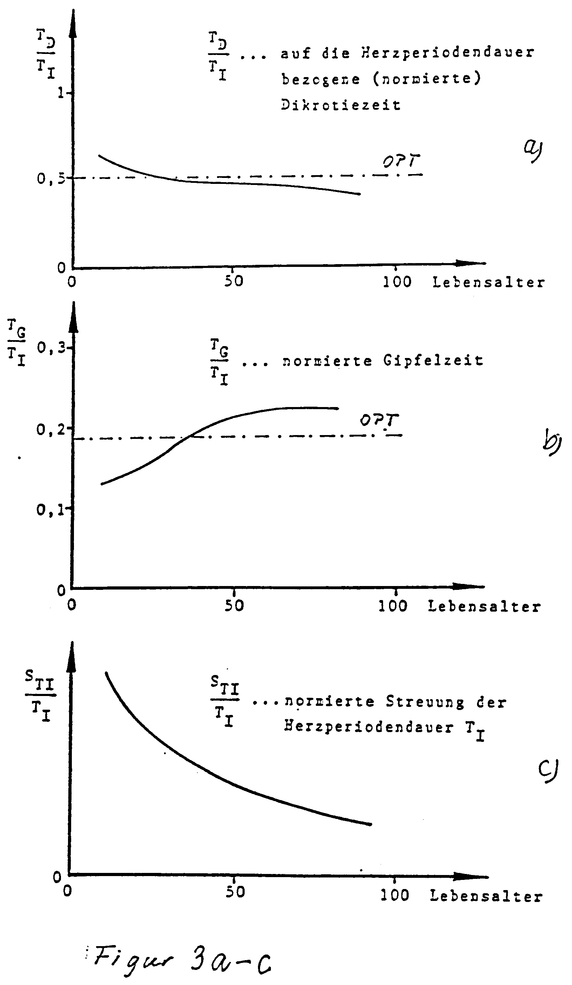

Mit den Figuren 3a bis 3c werden in Abhängigkeit vom Lebensalter die Normalwerte von normierten Parametern $T_D/T_I$, $T_G/T_I$, $S_{TI}/T_I$ sowie die Verhältnisse im Optimalfall OPT illustriert. Es ist anzumerken, daß es sich bei den Darstellungen um normierte Werte handelt. Mit dem Verhältnis $T_D/T_I$ wird die normierte Dikrotiezeit bezeichnet, mit dem Verhältnis $T_G/T_I$ die normierte Gipfelzeit und mit dem Verhältnis $S_{TI}/T_I$ die normierte Streuung der Pulsperiodenzeit.

Die Einordnung von Normalwerten ist deutlich zu erkennen. Die ausgezogenen Linien stellen jeweils die Mittelwertkurve ohne berücksichtigten Streubereich für den gesunden Menschen dar. Die strichpunktierte Linie in den Figuren 3a und 3b repräsentieren das theoretische Optimum OPT.

Vergleicht man nun tatsächlich ermittelte Werte mit den genannten, zweckmäßigerweise gespeicherten Mittelwertkurven, dann ist es in einfacher Weise möglich, daß der behandelnde Arzt gegebenenfalls typische Krankheitsbilder den tatsächlichen Werten zuordnen kann.

Durch die vorgeschlagene Messung der normierten Zeitverhältnisse und die Bestimmung der Streuung bei Berücksichtigung der normierten Amplitudenwerte können ansonsten auftretende Meßfehler eliminiert und die Auswertung dadurch vereinfacht werden.

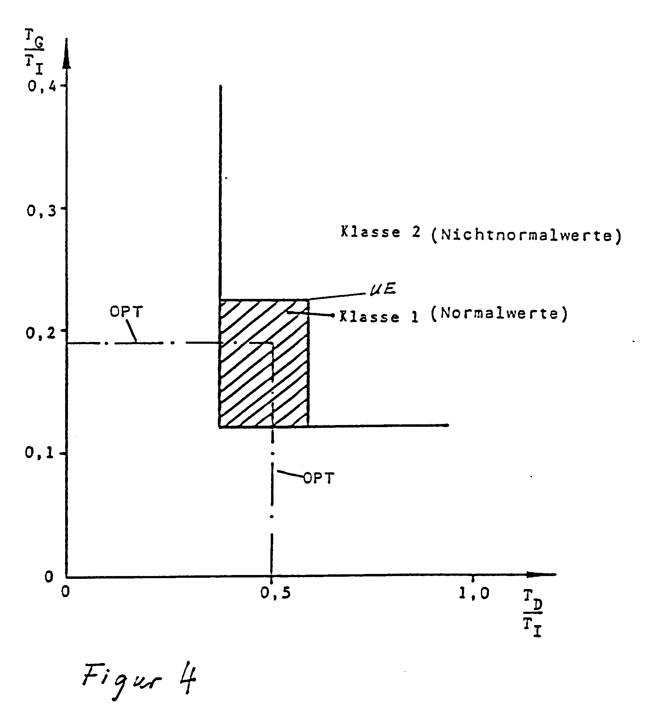

Zwecks Aufstellung von Bewertungskriterien wurden klinisch gesunde und kranke Probanden (im vorliegenden Fall mikroangioorganische Gefäßveränderungen bei Probanden mit Diabetes mellitus) untersucht. Es wird hierfür ein allgemeiner "Mikrogefäßzustand" für die Abhängigkeit der (normierten) Gipfelzeit $T_G/T_I$ von der (normierten) Diskrotiezeit $T_D/T_I$ definiert, wie Fig. 4 illustriert. Den erhaltenen Meßwerten werden die Klassen zugeordnet

- Klasse 1 (Normalwerte),

EP 0 600 298 A1

- Klasse 2 (Nichtnormalwerte, z.B. mikroangioorganische Gefäß veränderungen bei diabetischer Angiopathie).

Wenn Meßwerte im gekennzeichneten Übergangsbereich UE bestimmt werden, sollte eine Messung im Sinne der Erhöhung der Meßgenauigkeit wiederholt werden. Selbstverständlich ist die Bildung weiterer Unterklassen möglich.

Wie aus den Fig. 3a und 3b hervorgeht, verhalten sich die normierten Zeitverhältnisse $T_D/T_I$ und $T_G/T_I$ in Abhängigkeit vom Lebensalter gerade umgekehrt. Um diesen Alterseinfluß bei Bewertungskriterien nahezu auszugleichen, wird in einem Ausführungsbeispiel der Erfindung nach Fig. 4 ein "integraler Mikrogefäßzustand" $M_G$ durch Addition der normierten Parameter eingeführt, so daß ein (dimensionsloser) Wert entsprechender Größe erhalten wird:

$$M_G = T_G/T_I + T_D/T_I.$$

Mit den empirisch gefundenen Optima für $T_G/T_I$ und $T_D/T_I$ erhält man als optimalen Wert

$$M_{G\,opt} = 0,691,$$

während bei pathologischen Fällen (z.B. bei mikroangioorganischen Gefäßveränderungen) Werte von $M_G \approx$ 1 auftreten. Folglich ist auch hier eine Klassifizierung in Normalwerte (Klasse 1) und Nichtnormalwerte (Klasse 2) bei Auftreten eines Übergangsbereiches UE in einfacher Weise möglich.

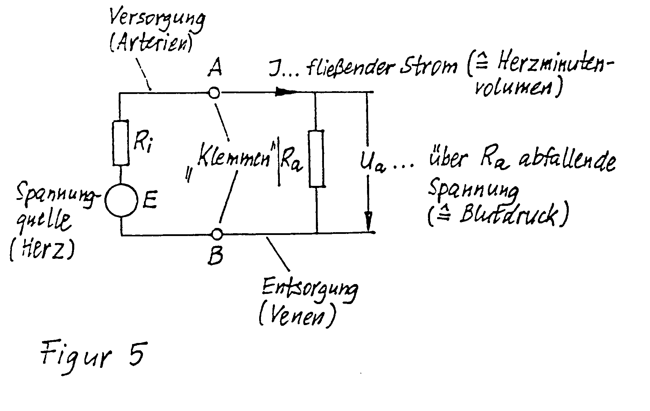

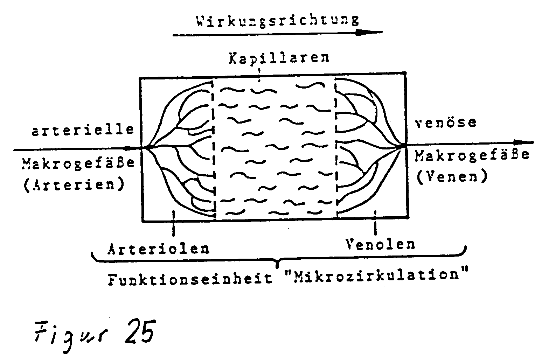

Mit einem weiteren komplexen Ausführungsbeispiel der Erfindung nach Fig. 5 soll gezeigt werden, daß sich der Blutkreislauf als Modell darstellen läßt, bei welchem eine Analogie zum elektrotechnischen Grundstromkreis im Sinne des Zusammenwirkens zwischen Spannungsquelle (Herz) und Peripherie $R_a$ - (Widerstandgefäße der Funktionseinheit Mikrozirkulation, wie Fig. 25 zeigt) über den Blutkreislauf betrachtet wird.

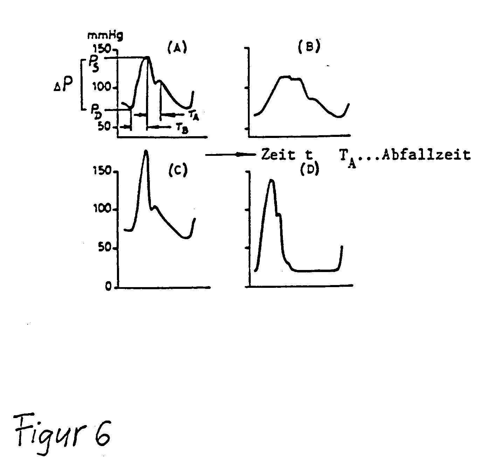

Aus Fig. 6 geht hervor, daß aus dem zentralen Puls neben der Amplitude die gleichwertigen charakteristischen Zeitkennwerte der systolischen Gipfelzeit $T_B$ sowie der Abfallzeit $T_A$ zwischen systolischem Gipfel und dikroter Einkerbung ableitbar sind, die ein Maß für den systolischen und diastolischen Blutdruck $P_S$, $P_D$ darstellen. Damit läßt sich erfindungsgemäß der relative periphere Widerstand $RPW_1$ als Ausdruck des im Grundstromkreis wirkenden Innenwiderstandes $R_i$ bestimmen.

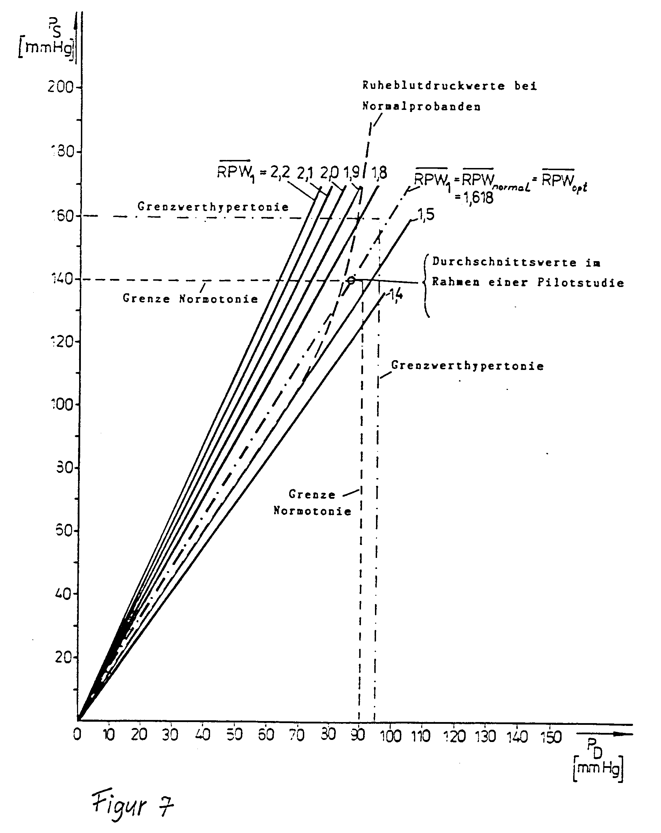

In Fig. 7 ist die Abhängigkeit zwischen systolischem und diastolischem Blutdruck $P_S$, $P_D$ mit verschiedenen Werten des relativen peripheren Widerstandes $RPW_1$ als Parameter, den Ruheblutdruck-Normalwerten einschließlich festgelegten Aussteuerungsgrenzen dargestellt. Es wird deutlich, daß der eingeführte Widerstand $RPW_1$ ein quantitatives Maß für das Zusammenwirken mit der Peripherie ist.

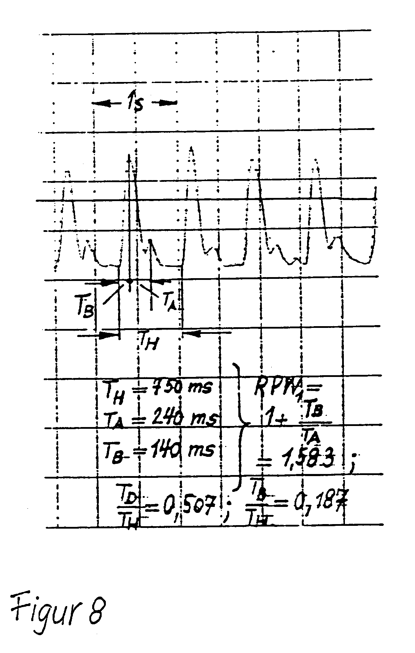

Fig. 8 zeigt dabei eine mit einem Ultraschall-Doppler aufgenommene Fließgeschwindigkeit in einer Armarterie, woraus die charakteristischen Zeitkennwerte $T_A$, $T_B$ nach Fig. 6 ebenfalls ableitbar sind.

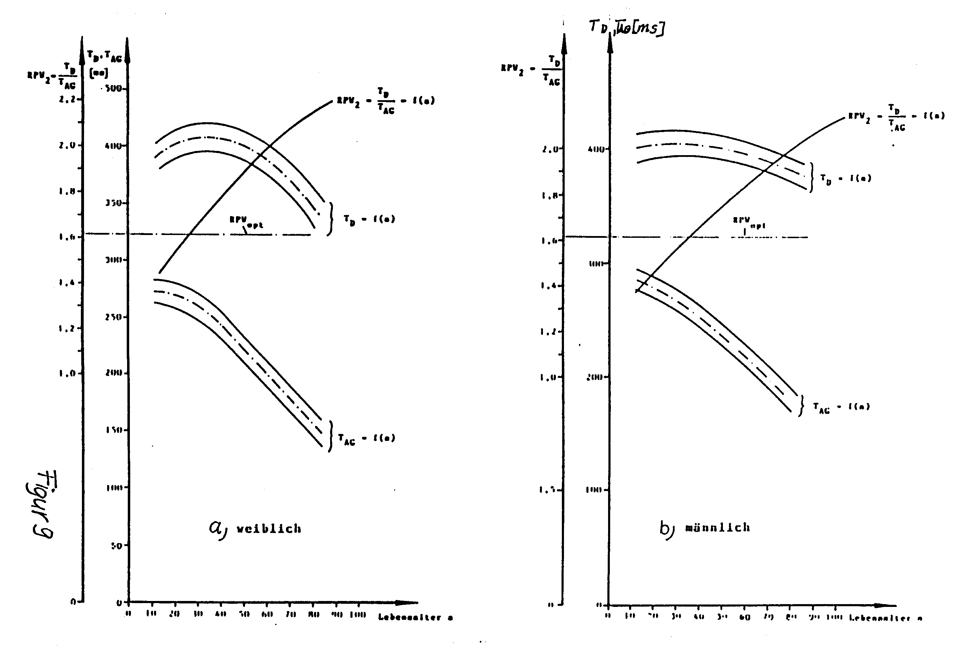

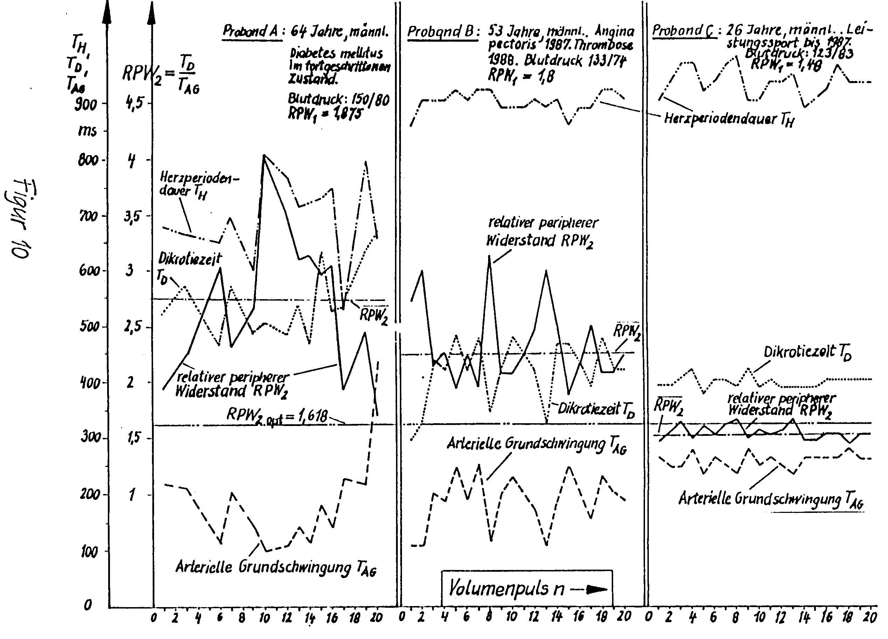

Mit der Fig. 9 wird die Abhängigkeit des erfindungsgemäß definierten relativen peripheren Widerstandes $RPW_2$, der dem Belastungswiderstand $R_a$ im Grundstromkreis entspricht und für jeden auftretenden Volumenpuls der Mikrozirkulation bestimmt werden kann, in Abhängigkeit des Lebensalters illustriert. Dabei sind ebenfalls die auf diesem Widerstand beruhenden Dikrotiezeiten $T_D$ sowie die arterielle Grundschwingung $T_{AG}$ aufgeführt.

Fig. 9a stellt die Verhältnisse bei weiblichen, Fig. 9b bei männlichen Probanden dar. Deutlich zeigt sich die Einordnung des ermittelten optimalen Widerstandes $RPW_{2\,opt} = 1,618$.

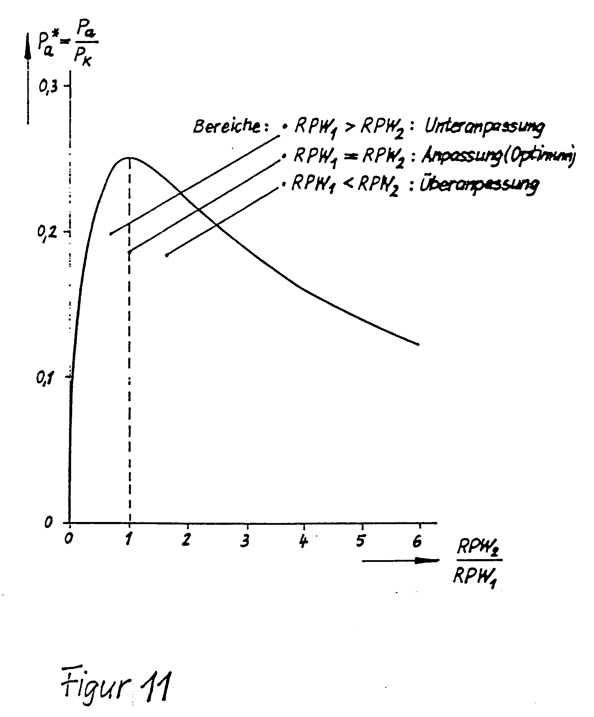

Fig. 10 zeigt die konkreten Meßdaten bei drei charakteristischen Probanden, wobei die Werte aus jedem gemessenen Volumenpuls der Mikrozirkulationsfunktionen bestimmt werden.

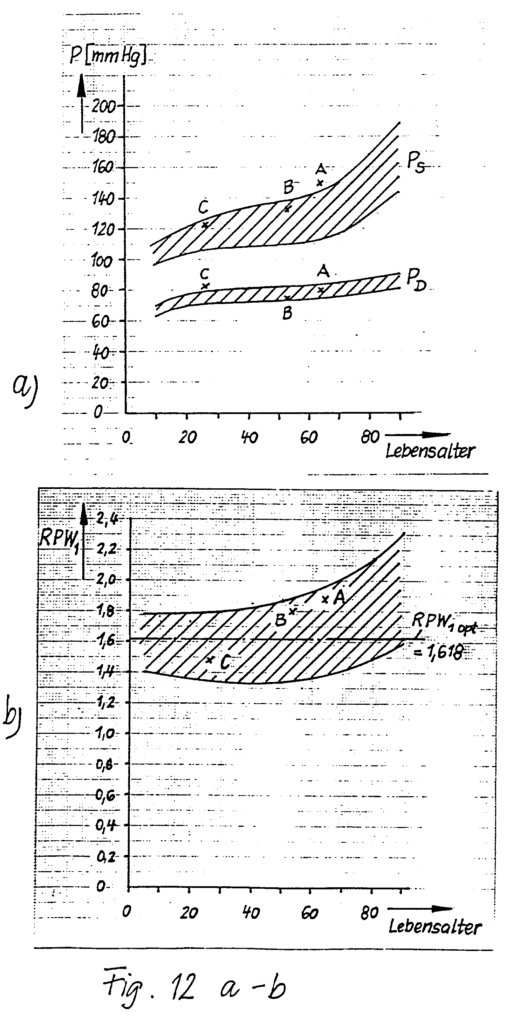

Mit der Fig. 11 wird das optimale Zusammenwirken zwischen Herz und Peripherie im Sinne von Fig. 5 deutlich. Bei $RPW_1 = RPW_2 = RPW_{opt}$ liegt erfindungsgemäß der Fall der Anpassung im Grundstromkreis vor, so daß ein Maximum an Leistung $P_a^*$ von der Quelle zum Verbraucher übertragen wird. Dabei stellt $P_a^*$ die normierte Verbraucherleistung dar (Verbraucherleistung $P_a$ wird auf die Leistungsabgabe $P_k$ des Generators bei Kurzschluß bezogen).

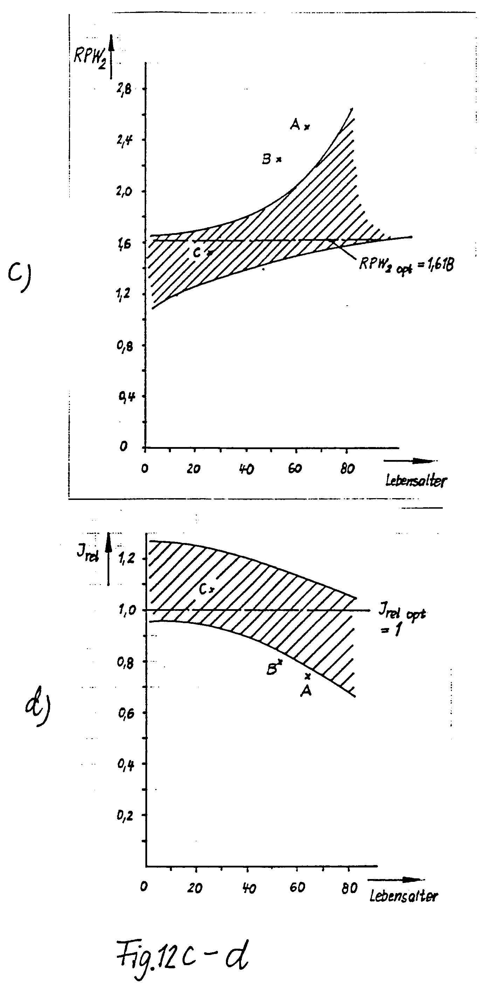

Mit Fig. 12 als weiteres Ausführungsbeispiel der Erfindung werden Normalbereiche in Abhängigkeit vom Lebensalter für den Ruheblutdruck (Fig. 12a), daraus abgeleitet für den relativen peripheren Widerstand $\overline{RPW_1}$ (Fig. 12b), für $\overline{RPW_2}$ (Fig. 12c) und für den relativen mittleren (Blut-) Stromfluß $I_{real}$ (Fig. 12d) angegeben. Als Beispiele A, B, C sind die Meßwerte für Probanden nach Fig. 10 eingezeichnet. Daraus geht hervor, daß nur Proband C im Normalbereich liegt.

In einem weiteren Ausführungsbeispiel der Erfindung werden zur Auswertung der erhaltenen Informationen aus der Mikrozirkulation die Autokorrelationsfunktionen gebildet.

11

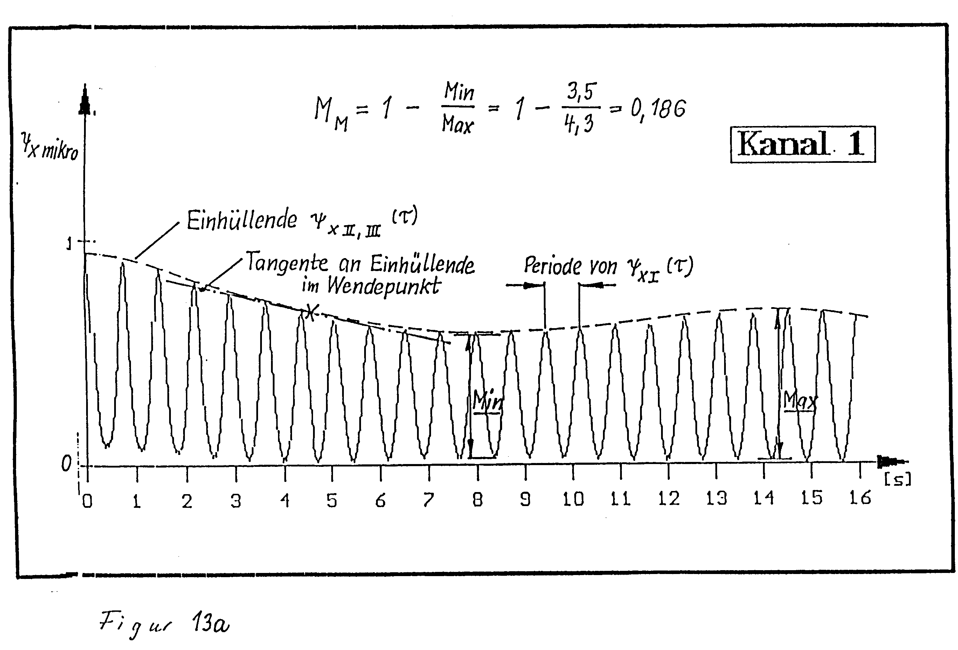

In der Fig. 13a und 13b werden Autokorrelationsfunktionen $\psi_{xges}(\tau) = \psi_{xmikro}(\tau)$ mit entsprechenden abgeleiteten Kenngrößen (13a) und mit stark ausgeprägter Atmungskomponente (13b) gezeigt.

Es können daraus folgende Rückschlüsse gezogen werden. Die Anteile der Teil-Autokorrelationsfunktionen hängen von den Anteilen der zugehörigen Zeitfunktionen $x_I(t)$, $x_{II}(t)$, $x_{III}(t)$ innerhalb von $x_{ges}(t)$ ab. Die Gesamt-Autokorrelationsfunktion stellt sich als eine Überlagerung der Teil-Autokorrelationsfunktionen dar. In Fig. 13a betragen die mittleren Perioden von $\psi_{xI}(\tau)$ ungefähr 0,75 s, die von $\psi_{xIII}(\tau)$ ungefähr 14 s; in Fig. 13b die Atmungskomponente ungefähr 5 s. Die Einhüllende von $\psi_{xges}(\tau)$ muß im Normalfall in einem bestimmten Zeitintervall abfallen und wieder ansteigen, wie die Fig. 13a und 13b zeigen. Als Maß wird ein sogenannter Mikrozirkulationsgradient $M_{mikro}$ aus $\psi_{xges}(\tau)$ abgeleitet.

$M_{mikro}$ ergibt sich aus folgender Beziehung:

$$M_{mikro} = 1 - Min/Max$$

wobei mit Max das Maximum der periodischen Funktion $\psi_{xges}(\tau)$ und mit Min ein zugehöriges Minimum definiert ist (Fig. 13a).

Analog zur Autokorrelationsfunktion ist eine Kreuzkorrelationsfunktion $\psi_{xy}(\tau)$ als Maß für die statistische Abhängigkeit zweier Funktionen x(t) und y(t) bestimmbar. Das Maximum der Kreuzkorrelationsfunktion tritt i. a. nicht bei $\tau = 0$ auf, sondern bei einer mittleren Laufzeit $\tau_{opt}$ zwischen den gewählten Meßstellen.

Wird die Kreuzkorrelationsfunktion $\psi_{xy}(\tau)$ bei der integralen Volumenpulsation gebildet, zum Beispiel am gleichen Finger mit den Meßstellen 1 und 2, und wird das Maximum bei $\tau = 0$ erhalten, sind die integrale Volumenpulsationen gleich.

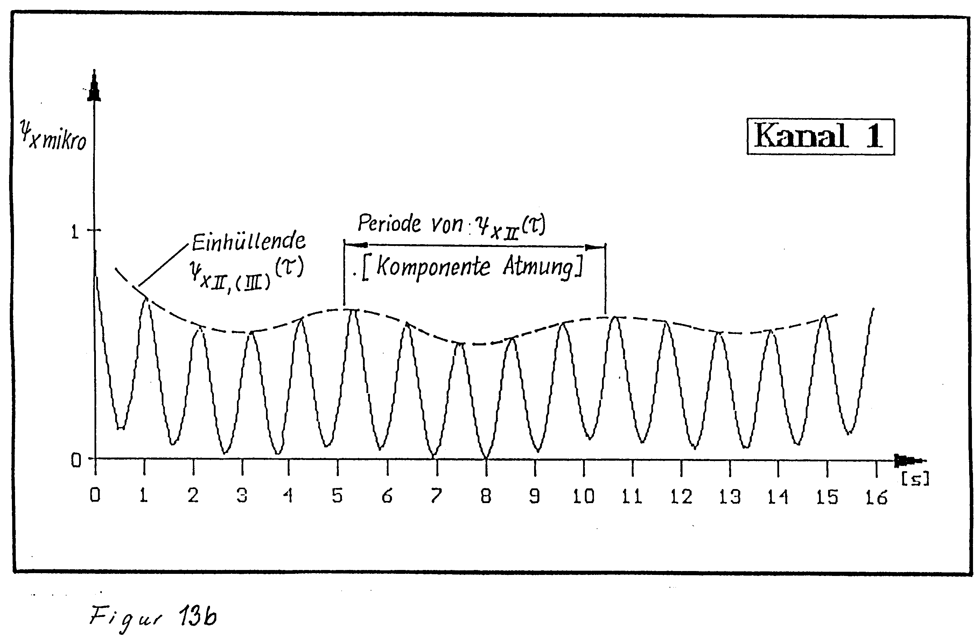

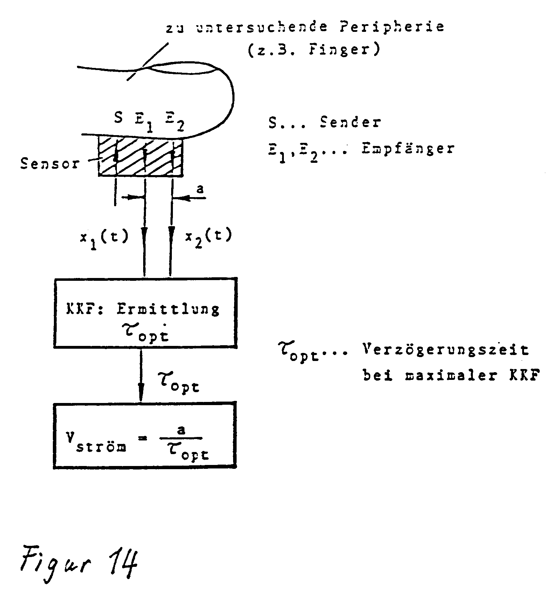

Mit der Fig. 14 wird illustriert, wie auf der Basis der Kreuzkorrelationsfunktion KKF die mittlere Blutströmungsgeschwindigkeit $v_{ström}$ zwischen zwei Meßstellen bestimmt werden kann. Die gezeigten Empfänger E1 und E2 liefern die vom Sender S ausgesendeten und im peripheren Gebiet reflektierten Signale $x_1(t)$ und $x_2(t)$. Die Kreuzkorrelationsfunktion bestimmt sich dann wie folgt:

$$\psi_{x1x2}(\tau) = \overline{x_1(t) \cdot x_2(t - \tau)}$$
$$= \psi_{x1x1}(\tau - T),$$

wobei T die mittlere Laufzeit darstellt, die das Blut braucht, um von Meßstelle E1 zu Meßstelle E2 zu gelangen. Da die Kreuzkorrelationsfunktion das Maximum bei $\tau_{opt} = T$ hat, gilt für die mittlere Blutströmungsgeschwindigkeit $v_{ström}$ zwischen den Meßstellen E1 und E2 folgende Beziehung:

$$V_{ström} = a/\tau_{opt};$$

mit a = Abstand zwischen E1 und E2.

In Ausgestaltung der Erfindung kann beispielsweise die Eingangsgröße an Meßstelle E1 stoßartig verändert werden, indem ein einmaliges, kurzes Husten erfolgt, wodurch ein quasi Nadelimpuls erzeugt wird, welcher sich von der Meßstelle E1 zur Meßstelle E2 fortpflanzt.

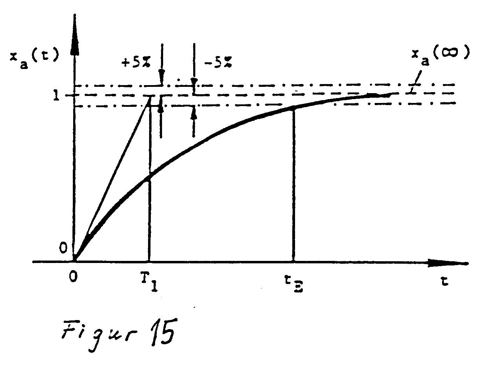

In der Fig. 15 wird die Definition der Einschwingzeit $t_E$ deutlich gemacht; $t_E$ ist die Zeit, nach der die Antwort $x_a(t)$ auf einen eingangsseitigen Sprung endgültig innerhalb der Schranken von $\pm$ 5% von $x_a(\infty)$ verbleibt. Für den Fall eines sogenannten negativen hydrostatischen Druckprungs am Eingang des Systems wird auf die gleiche Definition zurückgegriffen.

Analoge Aussagen gelten für eine Stoßfunktion und die zugehörigen Stoßübergangsfunktion.

Es hat sich dabei als vorteilhaft erwiesen, daß für den Erhalt einheitlicher, vergleichbarer Werte für die Einschwingzeiten bei positivem und negativem Sprung $\Delta t \geq t_E$ ist. Mit anderen Worten muß sich das System bzw. das untersuchte periphere Gebiet in einem eingeschwungenen Zustand befinden, wenn auf den negativen Sprung ein positiver Sprung folgt.

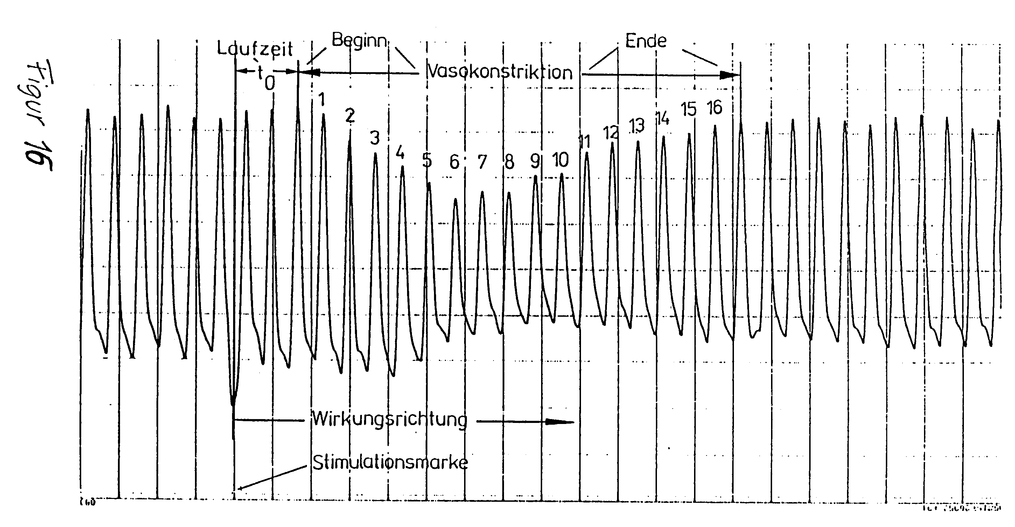

Wie in der Fig. 16 dargestellt, kann aus der Messung der konkreten Einschwingzeit $t_E$ des untersuchten peripheren Gebietes eine Beurteilung der Vasomotorik des Probanden erfolgen. Hierbei wird von der Tatsache ausgegangen, daß die Vasomotorik nicht von der Mikrozirkulation zu trennen und Teil des pulsierenden Systems ist. Es läßt sich also mit anderen Worten die Güte der Vasomotorik durch das Auslösen eines akustischen Reizes (Stoßfunktion) beurteilen. Die Antwort $x_a(t)$ hierauf stellt sich als leicht auszuwertende Stoßübergangsfunktion ein. Die Zeit zwischen Reizauslösung bis zum beginnenden Stoßübergang wird mit $t_0$ bezeichnet (Laufzeit). Die Güte der Vasomotorik kann durch die Aufnahme von $x_a(t)$

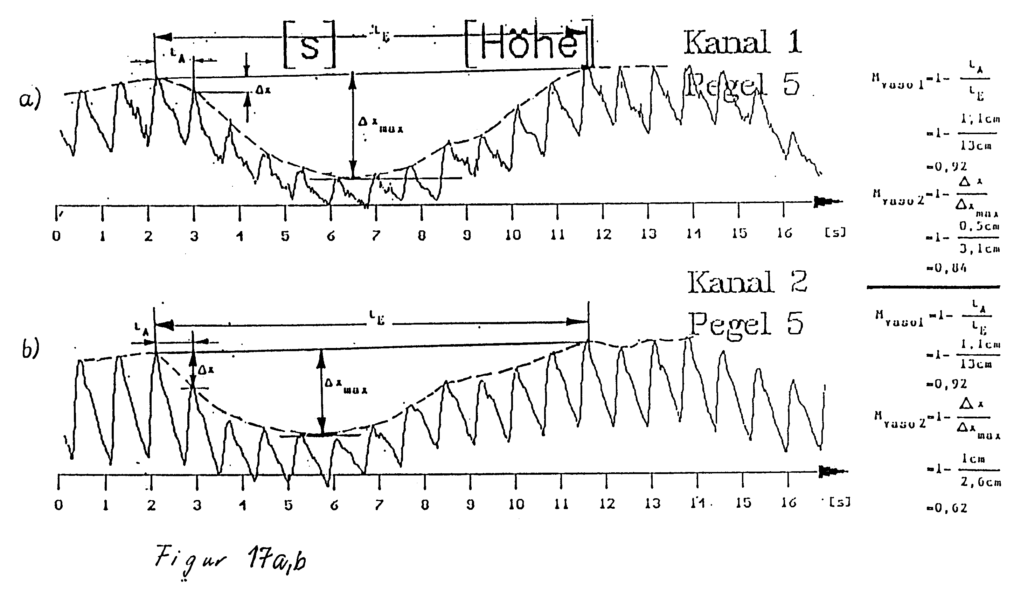

und der Einschwingzeit $t_E$ aus der Einhüllenden von $x_a(t)$ gemäß Fig. 17a, b erfolgen.

Bei fehlender Vasomotorik ergibt sich $t_E$ = 0. Fig. 17a zeigt vasomotorische Reaktionen bei einem Probanden gemessen am Finger und Fig. 17b die zeitgleich gemessenen Reaktionen an der Großzehe.

Durch die Auswertung der Zeitverhältnisse der Volumenpulsation kann eine erhöhte Meßgenauigkeit und damit eine verbesserte Auswertegenauigkeit erreicht werden. In diesem Sinne wird ein Vasomotorikgradient $M_{vaso\ 1}$ zur Beurteilung des jeweiligen Probanden eingeführt.

$M_{vaso\ 1}$ ergibt sich wie folgt:

$$M_{vaso\ 1} = 1 - t_A/t_E$$

mit

$t_A$ = Zeit zwischen zwei Schwingungsmaxima (Herzperiodendauer). Der ermittelte Faktor ist unabhängig von der Amplitude und besitzt die Grenzwerte:

1.) $t_A/t_E$ gegen 0, also $t_E$ sehr groß für $t_A$ gleich konstant: daher $M_{vaso\ 1}$ gegen 1.

2.) $t_E \leq t_A$, also keine oder eine geringe Reaktion im Intervall zwischen zwei Abtastungen: daher

$$M_{vaso\ 1} = 0...\ 0,5 \ (0,5 \ \text{für} \ t_E = t_A).$$

Die Einschwingzeit $t_E$ kann als Vielfaches n der Herzperiodendauer, d. h. der Anzahl der Volumenpulse, gesetzt werden. Es ergibt sich

$$t_E = n \cdot t_A.$$

Damit folgt:

$$M_{vaso\ 1} = 1 - t_A/n \cdot t_A$$

bzw.

$$M_{vaso\ 1} = 1 - 1/n.$$

Es ist also die Anzahl der einhüllenden Volumenpulse ein Maß für die Güte der Vasomotorik. Je mehr Volumenpulse von der vasomotorischen Reaktion erfaßt werden, umso mehr nähert sich $M_{vaso\ 1}$ dem Wert 1.

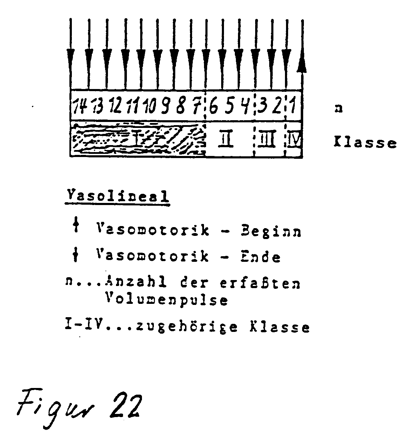

Durch die Zuordnung der Anzahl n der erfaßten Volumenpulse zu einer Klasse läßt sich eine Art Vasolineal konstruieren, wie in Fig. 22 gezeigt. Dieses Lineal beruht in Fig. 22 auf einer durchschnittlichen Herzperiodendauer von ca. 850 ms. Es wird an die Vasomotorik-Kurve angelegt und die Anzahl n sowie die Klasse werden abgelesen.

Auf der Basis dessen kann in einfacher Weise eine Klassifizierung in $M_{vaso\ 1}$ von sehr gut, gut, wenig Reaktion, keine Reaktion, vorgenommen werden.

Als zusätzliche Kenngröße kann gemäß Fig. 17a, b ein Vasomotorikgradient $M_{vaso\ 2}$ ermittelt werden.

$M_{vaso\ 2}$ ergibt sich aus der Beziehung wie folgt:

$$M_{vaso\ 2} = 1 - \triangle x/\triangle x_{max}$$

Hieraus erhält man:

$$0 \leq M_{vaso\ 2} < M_{vaso\ opt}.$$

Analog wie zu $M_{vaso\ 1}$ läßt sich im vorstehend definierten Bereich eine Klassifizierung durchführen.

Die Durchführung eines bekannten Bewegungsprogramms zur Aufnahme einer LRR-Kurve ist nicht bei allen Probanden möglich. Ebenfalls ist das Anbringen des Meßkopfes mittels Folienring zum Teil außerordentlich kompliziert. Die Entleerung der Gefäße erfolgt, ohne die Entleerzeit zu ermitteln. Es wird nur das Wiederauffüllen gemessen, wobei das Bestimmen der venösen Auffüllzeit oft stark fehlerbehaftet ist.

Im Sinne der Erfindung erweist sich folgende Verfahrensweise als deutlich vorteilhafter. Wegen der verbundenen Gefäße im zu untersuchenden peripheren Gebiet besteht ein unmittelbarer Zusammenhang bei Druckänderung zwischen den Makro- und Mikrogefäßen. Zur Ermittlung und Beurteilung des dynamischen Verhaltens des zu untersuchenden peripheren Gefäßgebietes wird folgender Systemtest durchgeführt.

Bei Raumtemperatur von ca. 22 bis 24°C wird ein Sensorkopf (z. B. mittels einer Klammer) beim In-Rücken-Ausgangslage befindlichen Patienten an der zu untersuchenden Geweberegion, z. B. an der Großzehe, positioniert. In dieser Position weist der Proband annähernd einen hydrostatischen Druck von 0 auf.

In horizontaler Lage wird ein funktionsdiagnostischer Sprung in Form eines negativen hydrostatischen Druckes angelegt. Das Bein oder der Arm des liegenden Probanden wird aktiv oder passiv möglichst schnell (Sprungfunktion) in eine konstante Endlage von ca. 30 bis 50 cm Höhe gebracht. Dabei entspricht die Sprunghöhe exakt der Höhe in cm Wassersäule des angelegten negativen, hydrostatischen Druckes. Das Vorgehen ist prinzipiell mit der Fig. 18 illustriert.

Durch einen negativen, hydrostatischen Sprung entleert sich das Gefäßsystem, es entsteht durch die Saugwirkung eine ständige Rückwirkung bis in den Kapillar- und arteriellen Bereich. Man erhält also eine Einschwing- oder Entleerzeit $t_{EL}$ des Mikro- und Makrogefäßsystems, die dort erreicht ist, wo der Entleervorgang einen konstanten Wert angenommen hat.

Folglich ist die Entleerzeit $t_{EL}$ ein direktes Maß für das dynamische Verhalten des untersuchten venösen Systems, zum Beispiel eines Beines.

Um vergleichbare Werte zu erhalten, muß für den Normalfall die Sprungdauer $\Delta t$ größer $t_{EL}$ sein, das System muß also eingeschwungen sein. Die Dauer des Sprunges sollte aus Erfahrungswerten 20 s nicht unterschreiten.

Nachfolgend wird nun ein Sprung in Form eines positiven hydrostatischen Druckes an das System angelegt. Hierfür wird das Bein möglichst schnell in die horizontale Ausgangslage zurück-gebracht.

Folglich wird die Einschwing- oder Auffüllzeit $t_{EF}$ (entspricht der venösen Auffüllzeit) der Mikro-Makrogefäße gemessen. Bei Venenklappen-Insuffizienz sind die Einschwingzeiten für das Entleeren und Füllen gleich groß.

Im Normalfall gilt mit ausreichender Näherung für den Zusammenhang zwischen Entleeren und Füllen $t_{EF}$ ungefähr gleich 1,6 $t_{EL}$.

Durch die Funktion der Venenklappen verlängert sich im Normalfall der Füllvorgang auf das ca. 1,6-fache im Vergleich zum Entleerungsvorgang.

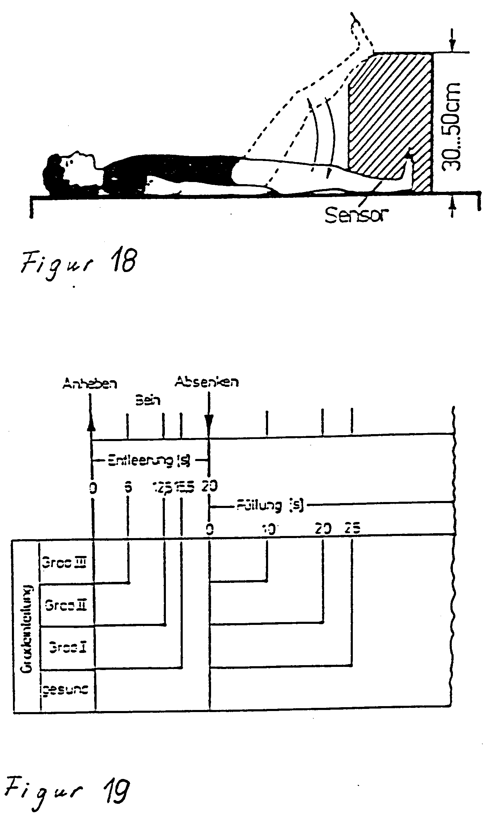

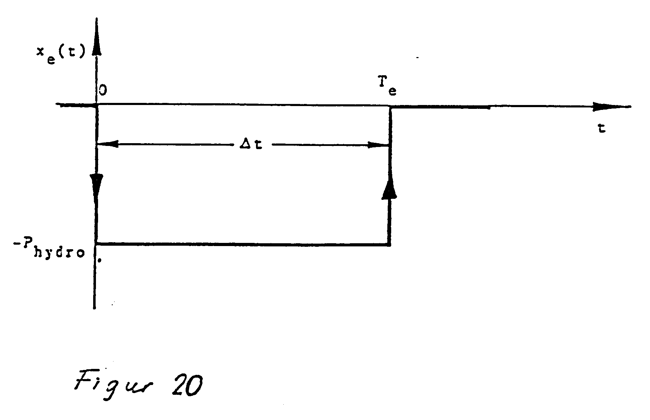

Die allgemeine hämodynamische Gradeinteilung für das Leeren und Füllen kann mittels eines sogenannten Gefäßzustandslineales nach Fig. 19, das unter Beachtung der Schreibgeschwindigkeit an eine aufgenommene Meßkurve angelegt werden kann, vorgenommen werden. Grundlage sind dabei für das Füllen die Normwerte der Phlebologie. Diese Werte lassen sich wegen des Zusammenhangs zwischen $t_{EL}$ und $t_{EF}$ auf das Entleeren erweitern und kennzeichnen allgemein den Mikro-Makro-Gefäßzustand. Fig. 20 zeigt die Systemeingangsgröße "hydrostatischer Druck".

Vorteilhafterweise wird der Systemtest aus Gründen der Vergleichbarkeit ca. zwei- bis dreimal hintereinander wiederholt.

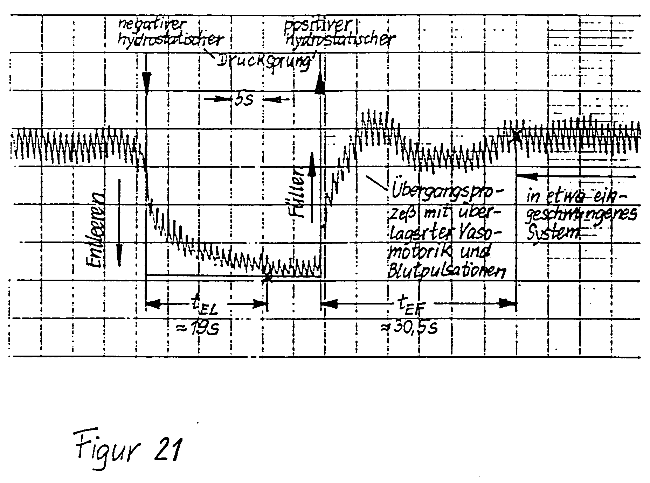

Mit der Fig. 21 sind praktische Ergebnisse eines Systemtests, wenn als Eingangsgröße sprungartig ein negativer und nachfolgend ein positiver hydrostatischer Druck (Entleeren und Füllen) angelegt wird, illustriert.

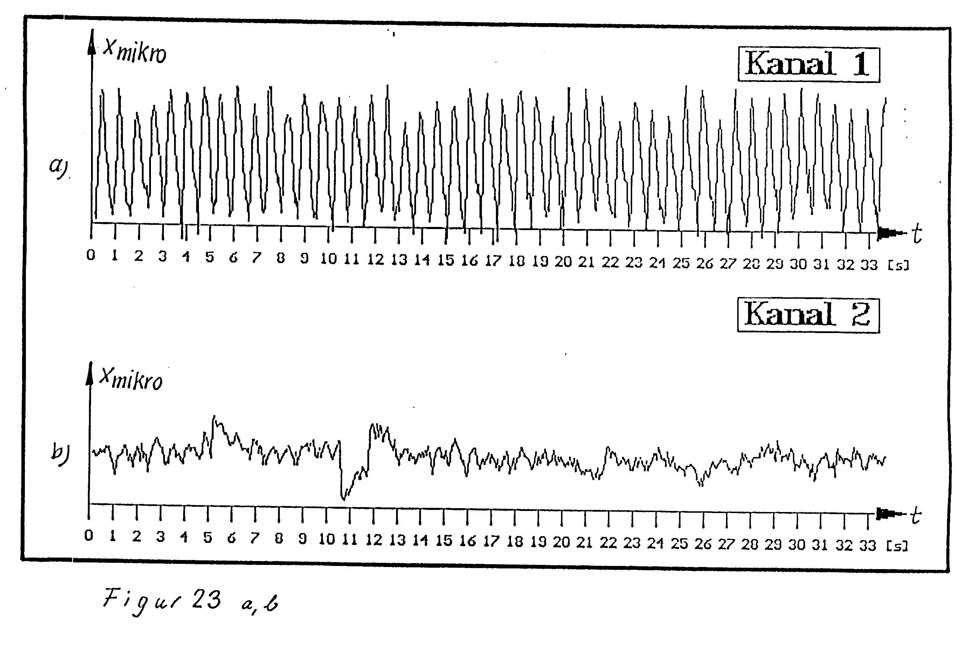

Mit der Fig. 23a und 23b wird eine simultane Messung der Mikrozirkulationsfunktion $x_{mikro}(t)$ bei einem Diabetiker im fortgeschrittenen Zustand gemäß a) am rechten Zeigefinger und b) an der rechten Großzehe illustriert.

Die Durchblutungsstörungen an der Zehe sind deutlich sichtbar, der Proband klagt über akute Gehbeschwerden im rechten Fuß.

Aus $x_{mikro}(t)$ nach Fig. 23a kann unter Anwendung der erfindungsgemäßen Lehre eine Berechnung und Darstellung u. a. folgender Werte erfolgen:

$T_D/T_I = 0{,}61$; $T_G/T_I = 0{,}389$; $T_I = 659$ ms; $M_G \approx 1$.

Diese Werte zeigen die starken mikroangioorganischen Gefäßveränderungen (nach Fig. 4 Klasse 2). Außerdem liegt die Herzperiodendauer $T_I$ deutlich unter Normalwert (zu hohe Herzfrequenz).

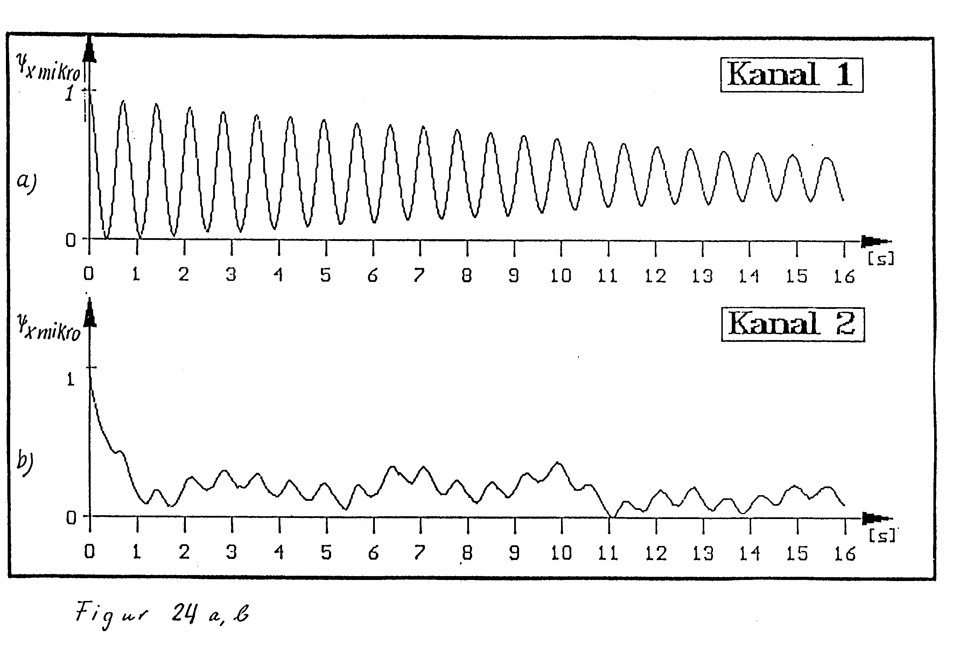

In den Figuren 24a und 24b sind die zugehörigen Autokorrelationsverläufe aus den Meßwerten der Figuren 23a und 23b dargestellt. Die AKF am Zeigefinger zeigt fallende Tendenz, woraus ersichtlich ist, daß die Mikrozirkulationsfunktion Biosignale enthält. In Fig. 24b wird deutlich, daß das Mikrozirkulationssignal einen großen nichtperiodischen Anteil (Bereich $0 < \tau < 1$ s), jedoch nur eine schwache periodische Mikrozirkulation als Ursachen der Gehbeschwerden enthält.

Aus dem Vorstehenden wird deutlich, daß das mit den Makrogefäßen verbundene Mikrogefäßsystem, d. h. die Funktionseinheit Mikrozirkulation, als komplexes Hoch- und Niederdrucksystem aufgefaßt werden kann.

Die Funktionseinheit Mikrozirkulation stellt sich, wie es in der Fig. 25 erläutert ist, als ein System mit einem arteriellen Zufluß, einem venösen Abfluß und einem Speicher dar.

Es läßt sich ableiten, daß für eine Geweberegion die in der Zeiteinheit zufließende Menge A gleich dem in der Zeiteinheit gespeicherten Mengenzuwachs B plus der in der Zeiteinheit abfließenden Menge C ist und damit eine dynamische Systemdiagnostik möglich ist (A = B + C).

Mittels einer simultanen Rot-NIR-Photoplethysmographie mit den Wellenlängen um 645 und 840 bzw. 940 nm, mit der jedoch keine an sich bekannte Oximetrie durchgeführt wird, lassen sich weitere Kenntnisse über das zu untersuchende Gefäßsystem erbringen.

Es wird im Sinne der Erfindung vorteilhaft ausgenutzt, daß die Photonen des Lichtes im roten Wellenlängenbereich relativ tief in das Gewebe eindringen können und entsprechend reflektiert werden, sofern dort einerseits sauerstoffangereichertes bzw. arterialisiertes Blut fließt und insofern dort andererseits gleichermaßen sauerstoffreduziertes bzw. venöses Blut fließt, welches in diesem Wellenlängenbereich entsprechend stark absorbiert. Diese Voraussetzungen treffen auf das ins Gewebe dreidimensional eingebettetes Mikrogefäßsystem zu, wobei mit der Remissions-Photoplethysmographie eine entsprechende Mikrozirkulationsuntersuchung stattfinden kann.

Erfindungsgemäß kann mittels des ins Gewebe eingeleiteten roten Lichtes in Folge der zu vernachlässigend geringen Speicherkapazität der Arteriolen, die weniger als 3% beträgt, gegenüber den venolären und venösen Kapazitätsgefäßen, auf die 75% (Venolen 12%, Venen 63%) des Blutvolumen-Fassungsvermögens entfallen, der arterielle Blutzufluß in die Arteriolen durch den unmittelbar dadurch bewirkten venösen Blutabfluß aus dem Mikrogefäßsystem bestimmt werden.

Demgemäß ist quasi eine direkte integrale Sicht auf das Mikrogefäßsystem der illuminierten Geweberegion möglich. Es lassen sich mit einem Ausführungsbeispiel gepulste NIR- und Rot-Strahlung einer mittleren Intensität im Multiplexbetrieb in das Gewebe einleiten, wodurch infolge der geringen Streuung und Absorption im Gewebe verhältnismäßig große Gewebevolumina ohne schädliche Rückwirkungen auswertbar sind.

Durch Messung des venösen Abflusses pro Zeiteinheit und des arteriellen Zuflusses ebenfalls pro Zeiteinheit läßt sich also der dynamische Systemzustand und damit auch die pro Zeiteinheit gespeicherte Menge ermitteln.

Eine praktische Realisierung kann gemäß einem Ausführungsbeispiel der Erfindung dadurch erfolgen, daß ein Doppelsensor, zum Beispiel an der Zehe oder einem Finger eines Probanden angebracht wird. Dieser Doppelsensor ermöglicht einen Multiplexbetrieb zur orts- und zeitgleichen, simultanen Rot- und NIR-Photoplethysmographie.

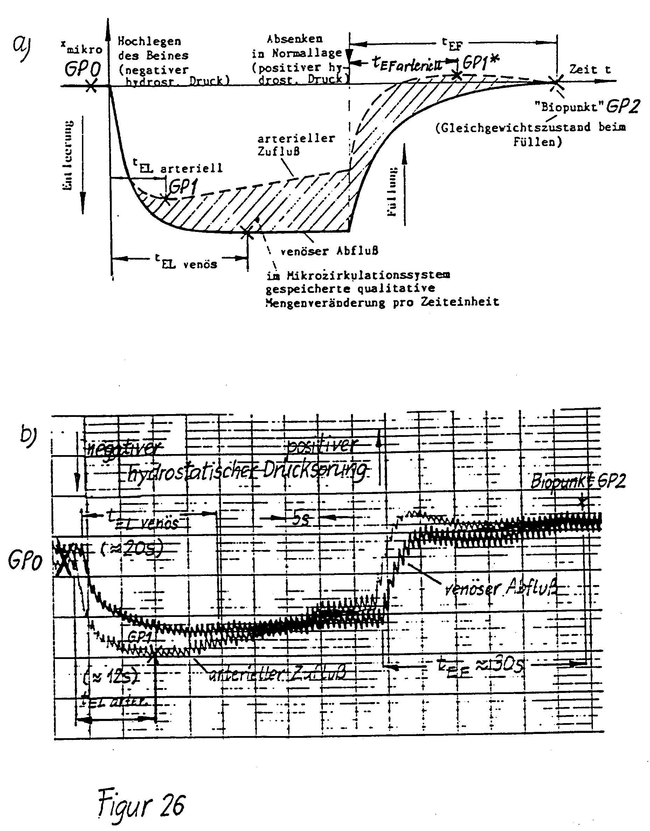

Wie bereits an anderer Stelle erwähnt, kann zur Ermittlung des dynamischen Systemzustandes eine Sprungfunktion in Form eines negativen sowie positiven hydrostatischen Druckes an die zu untersuchende Peripherie angelegt und das Leeren und Füllen der Funktionseinheit Mikrozirkulation und die sich ergebenden dynamischen Sprungübergangsfunktionen bestimmt werden. Hieraus lassen sich charakteristische Systemparameter ableiten, die eine einfache Auswertung des Zustandes ermöglichen, wie in Fig. 26a gezeigt wird. Die Fig. 26b stellt einen quantitativen Verlauf des arteriellen Zuflusses und venösen Abflusses innerhalb der Funktionseinheit der Mikrozirkulation, aufgenommen mit der vorerwähnten Realisierungsvariante eines Doppelsensors im Multiplexbetrieb, dar.

Aus dem Vorstehenden ergibt sich zusammenfassend, je komplexer das zu diagnostizierende System ist, desto einfacher und quantitativ reproduzierbarer das Auswerteverfahren sein muß. In diesem Sinne ist die an das Mikrogefäßsystem angelegte Testgröße hydrostatischer Druck besonders vorteilhaft. Diese Testgröße kann reproduzierbar, nichtinvasiv und quantitativ klar definiert angewendet werden. Der hydrostatische Druck als durch die Gravitationskraft bedingte Größe ist lediglich von der Höhe in zum Beispiel cm Wassersäule und der Fläche, auf der diese Wassersäule steht, abhängig.

Mit der simultanen Rot- und NIR-Remissions-Photoplethysmographie und Anlegen einer Sprungfunktion in Form negativen und positiven hydrostatischen Druckes kann festgestellt werden, was durch den angelegten Sprung im zu untersuchenden System an Veränderungen bewirkt wird und welche Größen im betrachteten System von der angelegten Sprungfunktion unberührt bleiben. Hieraus läßt sich eine hämodynamische Gradeinteilung für den arteriellen und venösen Bereich unter Zugrundelegung der Sprungübergangsfunktion in einfacher Weise ableiten. Im einzelnen kann die Beurteilung eines gestörten arteriellen Zuflusses aus den Makrogefäßen, zum Beispiel in Folge arteriosklerotischer Obliterationen ebenso wie Aussagen zum venosen Abfluß aus dem Mikrogefäßsystem einschließlich venöser Abflußstörungen in den Makrogfäßen, wie Venenthrombose, Venenklappeninsuffizienz, erfolgen.

Als weiteres Anwendungsgebiet kann die simultane Rot- und NIR-Photoplethysmographiezur differenzierten Beurteilung der Wirkung von Pharmaka oder Drogen auf das betrachtete Mikrogefäßsystem, zum Beispiel der vasomotorischen Reagibilität der Mikrogefäße, herangezogen werden.

Aus der hämodynamischen Gradeinteilung für die Bestimmung des Gefäßzustandes läßt sich noch nicht der Ort von Zufluß- oder Abflußstörungen im Makrobereich erkennen. Hierzu kann man das Prinzip der Bestimmung der Mikrozirkulation und die Methode der Sprungübergangsfunktion auf die Makrogefäße (Arterien, Venen) übertragen, indem die zeitliche Änderung der Strömungsgeschwindigkeit bei Anlegen eines entsprechenden hydrostatischen Druckes, zum Beispiel mittels dynamischer Ultraschall-Doppler erfaßt wird, also ebenfalls Kennzeitwerte daraus abgeleitet werden.

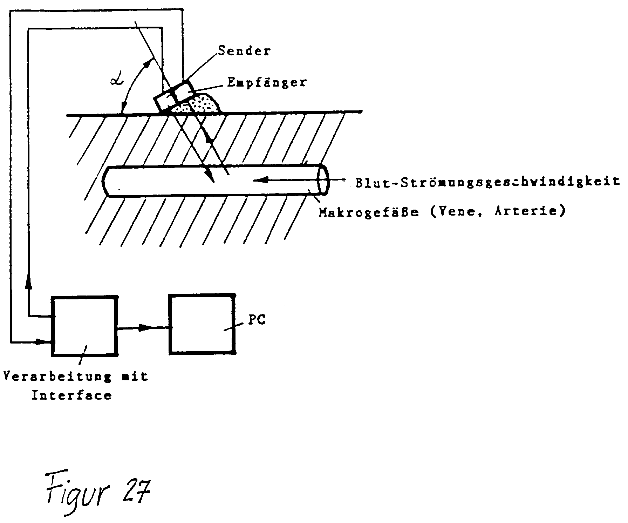

In Fig. 27 ist der prinzipielle Aufbau eines dynamischen Dopplers dargestellt, woraus ersichtlich ist, daß Sender/Empfänger wegen der minimalen Baugröße von der Verarbeitungselektronik zu trennen sind und in einem gesonderten, z. B.Klammer-Sensor-Gehäuse, integriert werden.

Demgemäß ergibt sich als weiteres Ausführungsbeispiel der Erfindung ein komplexes Verfahren mit folgenden Schritten. Zunächst wird bei konstanter Raumtemperatur der Klammer-Rot-NIR-Sensor beim In-Rücken-Ausgangslage befindlichen Patienten (z. B. an der Großzehe) positioniert. Dabei tritt dort annähernd ein hydrostatischer Druck von 0 auf.

Nachfolgend wird die Mikrozirkulation gemessen und ein negativer und positiver hydrostatischer Druck-Sprung angelegt. Aus dem Verlauf der Sprungübergangsfunktionen für das Entleeren und Füllen der Gefäße läßt sich nun feststellen, ob Normalwerte oder davon abweichende Verläufe vorliegen.

Wenn der Verlauf normal ist, kann die Auswertung beendet werden. Im Falle des Vorliegens von Abweichungen muß festgestellt werden, ob diese im Arteriellen oder Venösen liegen.

In diesem Falle wird erfindungsgemäß gezielt mit einem dynamischen Ultraschall-Doppler eine Fortsetzung der Untersuchung durchgeführt, indem der Doppler an der entsprechenden Meßstelle fixiert wird.

Die Strömungsgeschwindigkeitsmessung erfolgt nunmehr nicht, wie bekannt, rein stationär, sondern dynamisch. Mit dem dynamischen Doppler werden also die Sprungübergangsfunktionen und analog zur Mikrozirkulationsmessung die dynamischen Systemkennwerte des entsprechenden Makrogefäßes im Zeitbereich bestimmt. Hierfür wird ebenfalls ein negativer und positiver hydrostatischer Druck-Sprung angelegt, jedoch ist auch ein hydrodynamischer Drucksprung möglich.

Vorteilhafterweise wird bei der Untersuchung und Lokalisierung des Systems mit den größten Gefäßen (Vene, Arterie) begonnen, um den Aufwand gering zu halten.

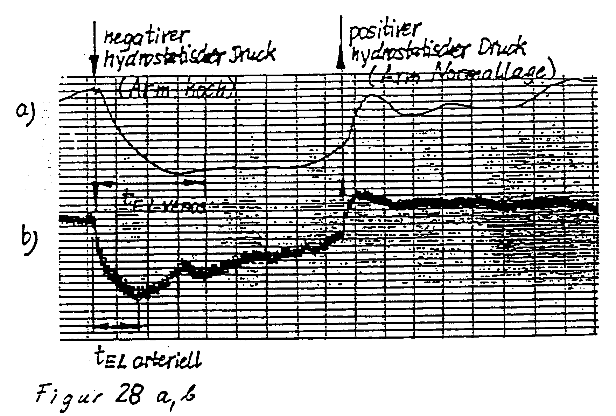

Die ermittelten Doppler-Sprungübergangsfunktionen im Makrobereich ähneln prinzipiell den Kurven, die bei der Rot- bzw. NIR-Photoplethysmographie im Mikrobereich erhalten werden. Es treten im arteriellen andere Übergangsfunktionen als im venösen Makrobereich auf, so daß auch sofort ersichtlich ist, ob eine Arterie oder eine Vene vorliegt und ob dabei der Verlauf normal oder nicht normal ist. Dies ist beispielhaft mit der Fig. 28 dargestellt, welche praktisch vorliegende Meßergebnisse für den linken Arm eines Probanden zeigt, wobei mit 28a eine mit einem dynamischen Ultraschall-Doppler aufgenommene venöse und mit 28b eine solche arterielle Sprungübergangsfunktion gezeigt ist.

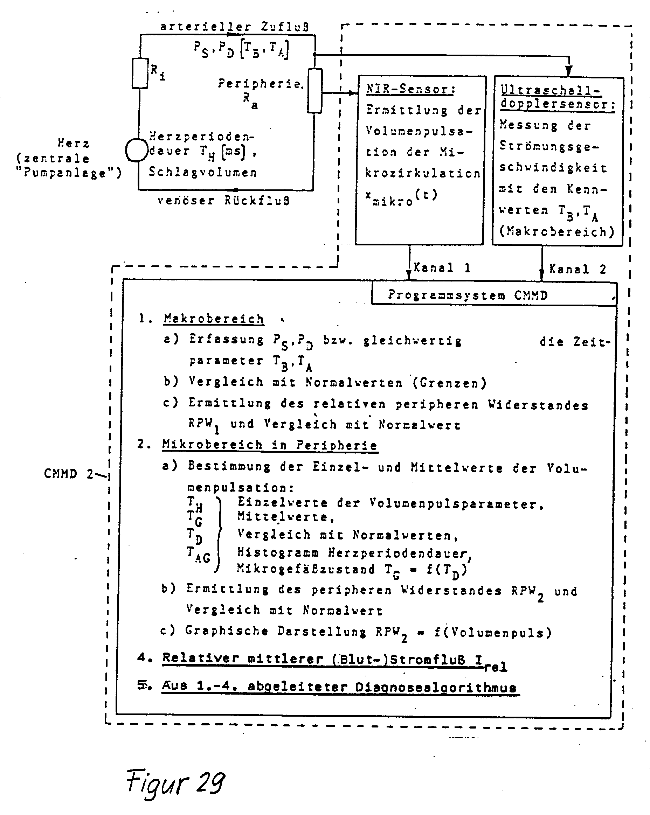

Fig. 29 zeigt ein Blockschaltbild einer Realisierungsform der Vorrichtung zur Gefäßzustandsbestimmung.

Im oberen Bildteil ist symbolisch das Modell des Grundstromkreises erläutert, wobei CMMD 2 eine computergestützte Einrichtung zur Bestimmung der Makro- und Mikrozirkulation des Gefäßsystems ist. Diese Einrichtung weist als Sensorikbaustein mindestens einen NIR-Sensor und einen Ultraschall-Dopplersensor auf. Die Meßergebnisse werden dann in getrennten Kanälen bereitgestellt und mit dem Programmsystem CMMD verarbeitet. Die wesentlichen Merkmale des Programmsystems sind mit den Ausführungen 1-4 im unteren Bildteil umrissen.

Mit dem erfindungsgemäßen Verfahren zur Bestimmung des Zustandes von Gefäßsystemen auf der Basis von Mikro- und Makrozirkulationsvorgängen wird erreicht, daß eine erhöhte Informationsmenge aus im wesentlichen unveränderten Ausgangsdaten bei allerdings verringerten apparativen Aufwand unter Vermeidung eines aufwendigen Bewegungsprogrammes erhalten werden kann. Durch die Erfindung wird die Informationsmenge so aufbereitet, daß für den diagnostizierenden Arzt eine einfache Zuordenbarkeit der erhaltenen Ergebnisse zu typischen Krankheitsverläufen oder Krankheitsbildern gegeben ist.

Das vorstehende Verfahren unterscheidet sich dabei grundsätzlich von bekannten Lösungen, welche auf einer Datenreduzierung oder dem bekannten Prinzip der Mustererkennung beruhen. Durch empirisch gefundene und statistisch determinierte Zusammenhänge wird eine einfache, übersichtliche Klassifikation der erhaltenen Ergebnisse möglich. Das erfindungsgemäße Verfahren ermöglicht es weiterhin, ausgehend von einer Grobklassifikation im Sinne einer Erweiterung der Diagnose in abgestufter Form zu einer Feinklassifikation überzugehen. Dem diagnostizierenden Arzt wird mit dem erfindungsgemäßen Verfahren die Möglichkeit gegeben, die vorhandene Informationsmenge selektiv, je nach konkreter Lage bzw. dem Zustand des Patienten zu erhöhen oder zu verringern. Bei Verwendung einer Recheneinrichtung bzw. einer Datenverarbeitungsanlage erfolgt dieses durch eine menuegestützte Aufbereitung eines hierarchisch aufgebauten Verzeichnisses von Auswertemodulen.

Dadurch, daß beim erfindungsgemäßen Verfahren die Erfassung von Zeit-Verhältniswerten bzw. deren Bestimmung im Vordergrund steht, ist eine Verallgemeinerung möglich. Ebensowenig ist eine Vorrichtung oder Einrichtung zur Steuerung eines rhythmischen Bewegungsablaufes erforderlich. Dies ist insbesondere dann von Vorteil, wenn der Patient aufgrund von Verletzungen oder anderen Ursachen nicht in der Lage ist, ein exakt vorgegebenes, relativ kompliziertes Bewegungsprogramm auszuführen. Hierfür sind insbesondere auch die stationären Messungen und deren Auswertung vorgesehen (z. B. bei mikroangioorganischen Gefäßveränderungen). Es wurde erkannt, daß das Zusammenwirken des Herzens mit der Peripherie einschließlich Blutkreislaufsystem durch die eingeführten relativen peripheren Widerstände und eine Näherungsbeziehung für den Sollwert der Herzperiodendauer beschreibbar ist.

Das erfindungsgemäße Verfahren ermöglicht also einerseits die Beschreibung des stationären Zustandes des zu untersuchenden Gefäßsystems auf der Basis der Bestimmung der Mikrozirkulation und/oder die Ermittlung des dynamischen Verhaltens des zu untersuchenden Systems durch Anlegen einer Sprungfunktion und Auswertung der Sprungübergangsfunktion.

Durch die Anwendung des Doppler-Prinzips in Verbindung mit der Auswertung der Sprungübergangsfunktion kann des weiteren der Ort einer möglichen Makrostörung erkannt werden. Ebenfalls können mit den stationären Dopplermessungen wegen der Auswertung von Zeitkennwerten im Echtzeitverfahren für jede Herzperiode die relativen Blutdruckwerte sowie der relative periphere Widerstand $RPW_1$ ermittelt werden.

Grundlegend für das erfindungsgemäße Verfahren ist das Prinzip der Messung und Untersuchung des Mikrosystems und die Auswertung von Zeitverhältnissen bzw. Zeitparametern, um Meßfehler weitgehend zu unterdrücken.

## Patentansprüche

1.  Arbeitsverfahren zum Betreiben einer Vorrichtung zur Bestimmung und Auswertung des Zustandes von Gefäßsystemen, insbesondere Blutgefäßen auf photoplethysmographischer Basis
    **gekennzeichnet durch**
    einen dynamischen Systemtest mittels:
    -   Beaufschlagen des zu untersuchenden Systems mit mindestens einer Sprung- oder Stoßfunktion $x_e(t)$;
    -   Erfassen der Übergangsfunktion $x_a(t)$, welche die Wirkung des zu untersuchenden Systems auf die Sprung- oder Stoßfunktion $x_e(t)$ darstellt;
    -   Ableiten von normierten Größen bzw. Kennwerten aus der Übergangsfunktion $x_a(t)$ durch Darstellung der Systemkennlinie $x_a$ über $x_e$ und Bestimmung der Streuung $S_T$ der erhaltenen Werte;
    -   Vergleich der erhaltenen normierten Größen bzw. Kennwerte sowie der Streuung $S_T$ dieser Werte mit vorbestimmten Normalkennlinien und
    -   Klassifizierung der Vergleichsergebnisse beispielsweise zur Feststellung der Wirkung von Pharmaka oder Drogen.

2.  Arbeitsverfahren nach Anspruch 1,
    **dadurch gekennzeichnet**,
    daß die Sprung- oder Stoßfunktion $x_e(t)$ für das zu untersuchende System bzw. periphere Gebiet durch kurzzeitige, einmalige druckmechanische Krafteinwirkung oder akustischen Reiz auf das System realisiert wird.

3.  Arbeitsverfahren nach Anspruch 1,
    **dadurch gekennzeichnet**,
    daß bei zu untersuchenden Gliedmaßen die Funktion $x_e(t)$ durch einmaliges Heben oder Senken des betreffenden Gliedmaßes auf der Basis eines veränderten hydrostatischen Druckes erfolgt.

4.  Arbeitsverfahren nach Anspruch 1,
    **dadurch gekennzeichnet**,
    daß bei der Darstellung der Systemkennlinie $x_a$ über $x_e$ sich ein typischer Arbeitspunkt in Abhängigkeit vom zu untersuchenden System einstellt, welcher als Vergleichswert herangezogen wird.

17

**5.** Arbeitsverfahren nach Anspruch 1,
**dadurch gekennzeichnet**,
daß aus der Bestimmung der Streubreite $S_T$ der Herzfrequenz durch Mikrozirkulationsbeobachtung des zu untersuchenden Systems bzw. in einem peripheren Gebiet dieses Systems auf die Leistungsfähigkeit und Funktion des Herzens geschlossen wird.

**6.** Arbeitsverfahren nach Anspruch 5,
**dadurch gekennzeichnet**,
daß bei Übergang von einem irregulären Muster der Herzfrequenz zu einem einfacheren Muster auf eine eingeschränkte Herzfunktion geschlossen wird.

**7.** Arbeitsverfahren nach einem der Ansprüche 1 bis 6,
**dadurch gekennzeichnet**,
daß die photoplethysmographisch ermittelte Gesamtfunktion $x_{ges}(t)$ wie folgt beschrieben wird:

$$xges(t) = x_I(t) + x_{II}(t) + x_{III}(t) + ... + x_N(t) = x_{mikro}(t)$$

mit

| | |
|---|---|
| $x_{ges}(t)$, $x_{mikro}(t)$: | integrale Gesamt-Volumenpulsation des photoplethysmographisch untersuchten peripheren Gebietes (Mikrozirkulationsfunktion) |
| $x_I(t)$: | Welle I. Ordnung, verbunden mit der Herzfrequenz als Trägerfrequenz der Volumenpulsation |
| $x_{II}(t)$: | Welle II. Ordnung, verbunden mit der Atmung |
| $x_{III}(t)$: | Welle III. Ordnung, verbunden mit der Blutdruckperiode (ca 10 s - Rhythmus) |
| $x_N(t)$: | Wellen N-ter Ordnung (bis hin zu mehrtägigen Schwankungen), |

wobei die Teilfunktionen den Gesetzen des determinierten Chaos unterliegen und einzelne Teilfunktionen aus der Gesamtfunktion ausgefiltert werden.

**8.** Arbeitsverfahren nach Anspruch 7,
**dadurch gekennzeichnet**,
daß die Teilfunktion $x_I(t)$ aus $x_{ges}(t)$ durch einen Bandpaß mit den Grenzfrequenzen 0,6 Hz < f < 9 Hz bestimmt wird.

**9.** Arbeitsverfahren nach Anspruch 8,
**dadurch gekennzeichnet**,
daß aus dem Verlauf von $x_I(t)$ eine Gipfelamplitude A, eine Dikrotieamplitude B, eine Gipfelzeit $T_G$, eine Dikrotiezeit $T_D$, eine arterielle Grundschwingung $T_{AG}$ und eine Pulsperiodenzeit $T_I$ sowie deren Streuung S bestimmt werden, wobei der jeweilige Arbeitspunkt aus den Normierungen $T_D/T_I$, $T_G/T_I$, $T_{AG}/T_I$, $T_{AG}/T_D$, $T_G/T_D$, $S/T_I$ bestimmt wird und ein Amplitudenverhältnis AV aus B/A ermittelt wird und dieser Arbeitspunkt mit einem vorbestimmten optimalen Arbeitspunkt verglichen und zur Aufwertung der Leistungsfähigkeit des Systems oder der Wirkung von Pharmaka oder Drogen vorgegebenen Klassen zugeordnet wird.

**10.** Arbeitsverfahren nach Anspruch 9,
**dadurch gekennzeichnet**,
daß ein optimales Amplitudenverhältnis AV bei 0,618 liegt und sich optimale Verhältnisse (Kennwerte, Arbeitspunkte) bei nichtvorhandenen pathophysischen Veränderungen wie folgt einstellen

$$T_D/T_I = 0,5; T_G/T_I = 0,191; T_{AG}/T_I = 0,309;$$
$$T_{TAG}/T_D = 0,618; T_G/T_D = 0,382.$$

**11.** Arbeitsverfahren nach Anspruch 7 bis 10,
**dadurch gekennzeichnet**,
daß die aus der in einem beliebigen peripheren Gebiet kontinuierlich gemessenen Mikrozirkulationsfunktion abgeleitete Herzperiodendauer $T_I$ (bzw. Herzfrequenz) einschließlich deren Streuung $S_T$ mit Normalwerten verglichen wird.

**12.** Arbeitsverfahren nach Anspruch 7 bis 11,
**dadurch gekennzeichnet**,
daß bei verringerter bzw. erhöhter Streubreite $S_T$ der Herzperiodendauer Nichtnormalwerte erkennbar sind.

**13.** Arbeitsverfahren nach Anspruch 7,
**dadurch gekennzeichnet**,
daß aus den Teilfunktionen eine Autokorrelationsfunktion $\psi x_{ges}(\tau)$ durch Feststellung der Anteile der Teilfunktionen an der Gesamtfunktion und der mittleren Perioden gebildet wird und zur Auswertung der Verlauf der Einhüllenden der Autokorrelationsfunktion betrachtet und aus diesem ein Mikrozirkulationsgradient $M_{mikro}$ nach der Beziehung:

$$M_{mikro} = 1 - Min/Max$$

mit
Max = Maximum der periodischen Funktion $\psi x_{ges}(\tau)$ und
Min = zugehöriges Minimum,
bestimmt und der erhaltene Wert $M_{mikro}$ mit typischen Werten für eine eingeschränkte Leistungsfähigkeit des Systems verglichen wird.

**14.** Arbeitsverfahren nach einem der Ansprüche 1 bis 13,
**dadurch gekennzeichnet**,
daß an zwei verschiedenen Meßstellen eines peripheren Gebietes die Funktionen $x_1(t)$ und $x_2(t)$ bestimmt, diese Funktionen einer Kreuzkorrelation nach der Beziehung

$$\varphi_{x1x2}(\tau) = \overline{x_1(t) \cdot x_2(t - \tau)}$$
$$= \varphi_{x1x1}(\tau - T),$$

mit
T = mittlere Laufzeit des Blutes
unterzogen, das Maximum $\tau_{opt}$ = T der Kreuzkorrelationsfunktion bestimmt und die Blutströmungsgeschwindigkeit $V_{ström}$ nach der Beziehung

$$V_{ström} = a/\tau_{opt};$$

mit a = Abstand der Meßstellen bestimmt werden.

**15.** Arbeitsverfahren nach Anspruch 1 und 7,
**dadurch gekennzeichnet**,
daß die Güte der Vasomotorik durch Aufnahme der Antwortfunktion $x_I(t)$ = $x_a(t)$ und Bestimmung der Einschwingzeit $t_E$ aus der Einhüllenden von $x_a(t)$ ermittelt wird, wobei als Einschwingzeit $t_E$ die Zeit definiert ist, nach der die Stoßantwort innerhalb eines Bereiches von ± 5% von $x_a(\infty)$ verbleibt.

**16.** Arbeitsverfahren nach Anspruch 15,
**dadurch gekennzeichnet**,
daß die Einschwingzeit $t_E$ aus dem Verlauf der Mikrozirkulationsfunktion, d. h. dem Verhältnis zwischen der Zeit $t_B$ zwischen dem Auslösen einer positiven und einer negativen Sprungfunktion und der Zeit $t_{E\ mikro}$, die von der negativen Sprungfunktion bis zur Beendigung des Mikrozirkulations-Einschwingvorganges vergeht, bestimmt wird.

**17.** Arbeitsverfahren nach Anspruch 15,
**dadurch gekennzeichnet**,
daß die Anzahl n der die Antwortfunktion $x_a(t)$ einhüllenden Volumenpulse ein quantitatives Maß für die Güte der Vasomotorik nach der Beziehung $M_{Vaso}$ = 1 - 1/n darstellt und dabei eine Klassifizierung vorgenommen wird.

**18.** Arbeitsverfahren zur Bestimmung und Auswertung des Zustandes von Gefäßsystemen, insbesondere Blutgefäßen auf photoplethysmographischer Basis,
**dadurch gekennzeichnet**,
daß vor einem dynamischen Sprungfunktions-Systemtest der stationäre Zustand durch Messung der Mikrozirkulation des Systems bestimmt wird.

**19.** Arbeitsverfahren zur Bestimmung und Auswertung des Zustandes von Gefäßsystemen, insbesondere Blutgefäßen auf photoplethysmographischer Basis,
**dadurch gekennzeichnet**,
daß an das zu untersuchende System eine Sprungfunktion in Form eines negativen hydrostatischen Druckes zur Entleerung der Gefäße des Systems angelegt wird, wobei aus der Antwortfunktion die Einschwing- oder Entleerzeit $t_{EL}$ des gesamten Systems bestimmt wird, und wobei die Entleerzeit $t_{EL}$ ein Maß für den dynamischen Zustand des Systems ist.

**20.** Arbeitsverfahren nach Anspruch 19,
**dadurch gekennzeichnet**,
daß zusätzlich nachfolgend eine Sprungfunktion in Form eines positiven hydrostatischen Druckes zur Füllung der Gefäße des Systems angelegt wird, wobei aus der Antwortfunktion die Einschwing- oder Auffüllzeit $t_{EF}$ gemessen wird und aus dem Vergleich zwischen der Entleerzeit und der (Wieder)-Auffüllzeit auf den Mikro- und Makrozustand des Systems geschlossen wird, wobei sich im Normalzustand für die venöse (Wieder-) Auffüllzeit im wesentlichen das 1,6-fache der Entleerzeit ergibt.

**21.** Arbeitsverfahren nach Anspruch 20,
**dadurch gekennzeichnet**,
daß zur Bestimmung des Systemzustandes die jeweiligen Sprungübergangsfunktionen betrachtet werden.

**22.** Arbeitsverfahren nach einem der Ansprüche 1 bis 21,
**dadurch gekennzeichnet**,
daß zur Bestimmung des Ortes von Störungen im Makro-Gefäßsystem die Sprungübergangsfunktion als örtliche Änderung der Strömungsgeschwindigkeit bei Anlegen einer hydrostatischen oder hydrodynamischen Druck-Sprungfunktion mittels Ultraschall-Doppler erfaßt und ausgewertet wird.

**23.** Arbeitsverfahren zur Bestimmung und Auswertung des Zustandes von Gefäßsystemen, insbesondere Blutgefäßen auf photoplethysmographischer Basis
**gekennzeichnet durch**
- eine Modellnachbildung des Blutkreislaufes auf der Basis eines Grundstromkreises mit Reihenschaltung einer Spannungsquelle E mit einem Innenwiderstand $R_i$ und einem Verbraucher $R_a$, wobei darstellen

$$R_i = RPW_1(n) = P_S(n)/P_D(n) = 1 + T_B(n)/T_A(n) \text{ mit}$$

$P_S$ = systolischer Blutdruck
$P_D$ = diastolischer Blutdruck
$T_B$ = systolische Gipfelzeit im Blutdruckverlauf
$T_A$ = Abfallzeit zwischen systolischem Gipfel und dikroter Einkerbung
n = n-te Herzperiode;
- aus der Modellbildung sich einstellender optimaler Anpassungsfall $R_i = R_a$ mit $\overline{RPW_1} = \overline{RPW_1}$ $_{opt}$ = 1,618 und
- das betreffende Gefäßsystem hinsichtlich der Abweichung von diesem Optimalwert bewertet wird, insbesondere mit den Normalbereichen $\overline{RPW_1}$ in Abhängigkeit vom Lebensalter verglichen wird.

**24.** Verfahren nach Anspruch 23,
**dadurch gekennzeichnet**,
daß im Echzeitbetrieb für jede Herzperiode n die Zeitkennwerte $T_B$ und $T_A$ mittels Ultraschall-Dopplerverfahren, die relativen Blutdruckwerte und der relative periphere Widerstand $RPW_1$ ermittelt werden und der Mittelwert $\overline{RPW_1}$ nach folgender Beziehung bestimmt wird:

$$\overline{RPW_1} = 1/N \sum_{n=1}^{N} RPW_1(n) \ .$$

25. Arbeitsverfahren zur Bestimmung und Auswertung des Zustandes von Gefäßsystemen, insbesondere Blutgefäßen auf photoplethysmographischer Basis,
**gekennzeichnet durch**
- eine Modellnachbildung des Blutkreislaufes auf der Basis eines Grundstromkreises mit Reihenschaltung einer Spannungsquelle E mit Innenwiderstand $R_i$ und einem Verbraucher $R_a$, wobei

$$R_a = RPW_2(n) = 1 + T_G(n)/T_{AG}(n) = T_D(n)/T_{AG}(n)$$

mit
$T_G$ = Gipfelzeit
$T_D$ = Dikrotiezeit
$T_{AG}$ = arterielle Grundschwingung einer Volumenpuls periode und
n = n-te Herzperiode
ist;
- aus der Modellbildung sich einstellender optimaler Anpassungsfall $R_i = R_a = \overline{RPW_2} = \overline{RPW_2}_{opt}$ = 1,618 und
- das betreffende Gefäßsystem hinsichtlich der Abweichung von diesem Optimalwert bewertet wird, insbesondere mit den Normalbereichen von $\overline{RPW_2}$ in Abhängigkeit vom Lebensalter verglichen wird.

26. Arbeitsverfahren nach Anspruch 25,
**dadurch gekennzeichnet**,
daß im Echtzeitbetrieb für jede Herzperiode der Mittelwert $\overline{RPW_2}$ wie folgt ermittelt wird:

$$\overline{RPW_2} = 1/N \sum_{n=1}^{N} RPW_2(n) \approx 1 + \overline{T_G}/\overline{T_{AG}} = \overline{T_D}/\overline{T_{AG}}.$$

27. Arbeitsverfahren nach Anspruch 24 bis 26,
**dadurch gekennzeichnet**,
daß als Optimalwert des betrachteten Gefäßsystems sich folgende Beziehung einstellt:

$$\overline{RPW_1} = \overline{RPW_2} = \overline{RPW_{opt}} = 1,618,$$

wobei beim Überschreiten vorgegebener Abweichungen vom vorstehenden Wert auf Einwirkungen von Pharmaka oder Drogen geschlossen wird.

28. Arbeitsverfahren nach Anspruch 27,
**dadurch gekennzeichnet**,
daß bei anormalen Erhöhungen der mittleren Widerstandswerte $\overline{RPW_1}$ und $\overline{RPW_2}$ z. B. frühzeitig auf entstehende Arteriosklerose und/oder Mikroangio-Organopathien geschlossen werden kann.

29. Arbeitsverfahren nach einem der Ansprüche 23 bis 28,
**dadurch gekennzeichnet**,
- daß der im Blutkreislauf (Grundstromkreis) fließende relative mittlere (Blut-) Strom $I_{rel}$ sich dnach der Beziehung

$$I_{rel} = 3,236/ [\overline{RPW_1} + \overline{RPW_2}]$$

21

EP 0 600 298 A1

einstellt, wobei das Optimum $I_{rel\ opt}$ = 1 verläuft,

- daß sich Normalbereiche für $I_{rel}$ in Abhängigkeit vom Lebensalter mit den Normalbereichen $\overline{RPW_1}$ und $\overline{RPW_2}$ ergeben und somit eine Klassierung in Normal- und Nichtnormalwerte möglich ist.

**30.** Arbeitsverfahren nach einem der Ansprüche 23 bis 29,
**dadurch gekennzeichnet,**
daß sich beim optimalen mittleren peripheren Widerstandswert $\overline{RPW_{opt}}$ = 1,618 als Soll-Wert für die Herzperiodendauer $T_{H\ SOLL}$ [ms] ≈ 508 [1 + 1/$\overline{RPW_1}$] ≈ 508 [1 + $P_D/P_S$] einstellt, wobei Abweichungen vom Soll-Wert für die Herzperiodendauer $T_{H\ SOLL}$ im Falle des annähernden Übereinstimmens der peripheren Widerstandwerte mit dem optimalen pheripheren Widerstandswert zur weiteren Bewertung des Gefäßsystems herangezogen werden.

**31.** Arbeitsverfahren nach Anspruch 23 bis 30,
**dadurch gekennzeichnet,**
daß als indirekte Parameter für die laufende Überwachung und Einschätzung der Herz-Kreislauf-Situation einschließlich Auftreten von Hypertonie folgende Größen bewertet werden:
- $\overline{RPW_1}$ und $\overline{RPW_2}$
- absolute Blutdruckwerte $P_S$ und $P_D$ und/oder die Zeitkennwerte $T_A$ und $T_B$
- Herzperiodendauer $T_H$
- Druckanstiegs- und Abfallwerte je Zeiteinheit und
- der Mikrogefäßzustand.

**32.** Arbeitsverfahren nach Anspruch 31,
**dadurch gekennzeichnet,**
daß die relativen peripheren Widerstände vom Makro- und Mikrobereich des betrachteten Gefäßsystems $\overline{RPW_1}$ und $\overline{RPW_2}$ auf nichtinvasiver Basis bestimmt werden.

**33.** Vorrichtung zur Bestimmung und Auswertung des Zustandes von Gefäßsystemen, insbesondere Blutgefäßen auf photoplethysmographischer Basis, unter Verwendung eines lichtaussendenden und lichtempfangenden Sensorkopfes zur nichtinvasiven optischen Messung der Blutentleerung und Blutauffüllung der zu untersuchenden Gefäße bzw. des Gefäßsystems eines peripheren Gebietes zur Verwendung bei Tauglichkeitsuntersuchungen oder der Bewertung des Einflusses von Pharmaka und Drogen,
**dadurch gekennzeichnet,**
- daß mittels eines NIR-Sensors eine Ermittlung der Volumenpulsation der Mikrozirkulation $X_{mikro}(t)$ und mittels eines Ultraschall-Dopplersensors eine Messung der Strömungsgeschwindigkeit des Blutes mit den Kennwerten $T_B$ = systolische Gipfelzeit im Blutdruckverlauf und $T_A$ = Abfallzeit zwischen systolischem Gipfel und dikroter Einkerbung im Makrobereich erfolgt und
- die jeweiligen Meßwerte einer Einrichtung zur Bestimmung des Zusammenwirkens von Herz, Blutkreislauf und Peripherie zugeführt werden, wobei diese Einrichtung vorteilhafterweise mittels programmtechnischen Bestandteilen im Makrobereich eine Erfassung von $P_S$ = systolischen und $P_D$ = diastolischen Blutdruck bzw. der Zeitparameter $T_B$ und $T_A$, einen Vergleich dieser Werte mit Normalwerten und eine Ermittlung des relativen peripheren Widerstandes $\overline{RPW_1}$ einschließlich eines Vergleiches mit einem Optimalwert durchführt und
im Mikrobereich Einzel- und Mittelwerte der Volumenpulsation bestimmt sowie diese mit vorgegebenen Normal- bzw. Optimalwerten vergleicht, ein Histogramm der Herzperiodendauer erstellt und eine Bewertung des Mikrogefäßzustandes $T_G$ = $f(T_D)$ vornimmt;
- die Einrichtung eine Ermittlung des peripheren Widerstandes $\overline{RPW_2}$ durchführt und diesen Wert mit einem Optimalwert vergleicht sowie eine graphische Darstellung des peripheren Widerstandes $\overline{RPW_2}$ je Volumenpuls nach der Beziehung $\overline{RPW_2}$ = f (Volumenpuls) ermöglicht; und
- weiterhin eine Bestimmung des mittleren Blutdruckanstieges und -abfalls je Zeiteinheit mit anschließender Bewertung auf der Basis des Vergleiches der Druckanstiegs- und Druckabfallswerte je Zeiteinheit durchführt, wobei beim Vorliegen von Nichtnormalwerten, d. h. unterschiedlichen Druckanstiegs- und Druckabfallswerten, auf das Vorliegen oder Nichtvorliegen vorgegebener Kriterien geschlossen wird und
- daß aus den mittels der Einrichtung bestimmten Werten eine komplexe Analyse des Gefäßsystems durchgeführt wird, insbesondere die Ermittlung von Normalwerten für den relativen mittleren (Blut-) Stromfluß möglich ist.

**34.** Vorrichtung nach Anspruch 33,
**dadurch gekennzeichnet**,
daß eine Bewertung des Vorliegens vorgegebener Kriterien bzw. des Zustandes des Gefäßsystems mit nachstehenden Kriterien durchgeführt wird:

a) $\overline{RPW_1} \approx \overline{RPW_2} \approx \overline{RPW_{opt}}$;
$T_H$ im Sollwertbereich und $P_S$ und $P_D$ bzw. die gleichwertigen Zeitparameter $T_B$ und $T_A$ im Normalbereich:
normaler Gefäßzustand
b) $\overline{RPW_1} \approx \overline{RPW_2} \approx \overline{RPW_{opt}}$, $T_H$ = Normalwert, $P_S$ und $P_D$ im Nichtnormalbereich, z. B. Grenzwerthypotonie:
Schlagvolumen im Gefäßsystem zu hoch
c) $\overline{RPW_1} \approx \overline{RPW_2} \approx \overline{RPW_{opt}}$, $T_H$ < Normal- oder Sollwert, $P_S$ und $P_D$ im Nichtnormalbereich:
Herzperiodendauer zu klein, Herzfrequenz im Gefäßsystem zu hoch;
d) $\overline{RPW_1} > \overline{RPW_2} \approx \overline{RPW_{opt}}$:
zusätzliche Prüfung der Herzperiodendauer $T_H$ erforderlich, wobei eventuell zu hohe Herzfrequenz im betrachteten Gefäßsystem vorliegt, ebenfalls Venenpumpe überprüfen;
e) $\overline{RPW_1} < \overline{RPW_2} \approx \overline{RPW_{opt}}$:
Herzperiodendauer $T_H$ ist zu hoch bzw. Venenpumpe nicht optimal eingestellt.

**35.** Vorrichtung zur Bestimmung und Auswertung des Zustandes von Gefäßsystemen, insbesondere Blutgefäßen auf photoplethysmographischer Basis, unter Verwendung eines lichtaussendenden und lichtempfangenden Sensorkopfes zur nichtinvasiven optischen Messung der Blutentleerung und Blutauffüllung der zu untersuchenden Gefäße bzw. des Gefäßsystems eines peripheren Gebietes,
**dadurch gekennzeichnet**,
daß der venöse Abfluß pro Zeiteinheit und der arterielle Zufluß pro Zeiteinheit mittels simultaner Rot- und NIR-Photoplethysmographie gemessen werden, wodurch der dynamische Gefäß-Systemzustand und damit die pro Zeiteinheit gespeicherte Blutmenge bestimmbar sind.

**36.** Vorrichtung zur Bestimmung und Auswertung des Zustandes von Gefäßsystemen, insbesondere Blutgefäßen auf photoplethysmographischer Basis, unter Verwendung eines lichtaussendenden und lichtempfangenden Sensorkopfes zur nichtinvasiven optischen Messung der Blutentleerung und Blutauffüllung der zu untersuchenden Gefäße eines peripheren Gebietes zur Verwendung bei Tauglichkeitsuntersuchungen oder der Bewertung des Einflusses von Pharmaka und Drogen,
**gekennzeichnet durch**

- Mittel zum Beaufschlagen des zu untersuchenden Systems mit mindestens einer Sprungfunktion $x_e(t)$;
- Mittel zum Erfassen der Sprungübergangsfunktion $x_a(t)$ durch nichtinvasive optische Messung, wobei die Reaktion des zu untersuchenden Systems auf die Sprungfunktion $x_e(t)$ bestimmt wird;
- Mittel zum Speichern und Darstellen der Systemkennlinie $x_a$ über $x_e$ und Bestimmung einer Streuung $S_T$ der erhaltenen Werte sowie zur Bestimmung von typischen normierten Kennwerten des Systems;
- Mittel zum Vergleich der erhaltenen normierten Kennwerte sowie der Streuung $S_T$ dieser Werte mit vorbestimmten Normalkennlinien und zur Klassifizierung der Vergleichsergebnisse.

**37.** Vorrichtung nach Anspruch 36,
**dadurch gekennzeichnet**,
daß die Mittel zum Beaufschlagen des Systems mit der Sprungfunktion $x_e(t)$ eine kurzzeitige, einmalige druckmechanische Krafteinwirkung oder einen akustischen Reiz auf das System erzeugen.

**38.** Vorrichtung nach Anspruch 36,
**dadurch gekennzeichnet**,
daß bei zu untersuchenden Gliedmaßen Mittel zum einmaligen Heben oder Senken des betreffenden Gliedmaßes zur Veränderung des hydrostatischen Druckes vorgesehen sind.

**39.** Vorrichtung zur Bestimmung und Auswertung des Zustandes von Gefäßsystemen, insbesondere Blutgefäßen auf photoplethysmographischer Basis, unter Verwendung eines lichtaussendenden und lichtempfangenden Sensorkopfes zur nichtinvasiven optischen Messung der Blutentleerung und Blutauffüllung der zu untersuchenden Gefäße eines peripheren Gebietes

**dadurch gekennzeichnet**,

- daß die Mikrozirkulationsfunktion des Systems als integrale Gesamt-Volumenpulsation $x_{ges}(t)$ über die Zeit bestimmt und diese Funktion durch Bandpaßfilter in Wellen I. bis N-ter Ordnung zur Bestimmung der Teilfunktion $X_I(t)$ bis $x_N(t)$ zerlegt wird sowie mindestens die Teilfunktion $x_I(t)$, welche mit einem Bandpaß einer Grenzfrequenz 0,6 Hz < f < 9 Hz bestimmt ist, in geeigneter Weise gespeichert und dargestellt wird und aus dem Verlauf der Teilfunktionen $x_I(t)$ nach deren Zeitnormierung Merkmalsvektoren bestimmt werden;

- daß Mittel zum Erzeugen einer positiven und/oder negativen Sprungfunktion $x_e(t)$ auf der Basis einer kurzzeitigen akustischen Reizeinwirkung oder einer druckmechanischen Kraft auf das System vorgesehen sind und die das dynamische Systemverhalten repräsentierende Sprung-übergangsfunktion $x_a(t)$ mindestens auf der Basis der Mikrozirkulationsteilfunktion $x_I(t)$ ermittelt wird, wobei der dynamische Zustand der Makrozirkulation des Gefäßsystems aus dem Verlauf der Mikrozirkulationsfunktion bestimmbar ist; und

- daß der Sensorkopf derart ausgebildet ist, daß Strahlung einer Wellenlänge von 635 und 840 bzw. 940 nm simultan emittiert und empfangen wird, wodurch die sauerstoffangereicherte arterialisierte und sauerstoffreduzierte, venöse Blutzirkulation der im Gewebe des zu untersuchenden Systems eingebetteten Mikrogefäße ermittelbar ist und die Bestimmung des venösen Abflusses pro Zeiteinheit sowie des arteriellen Zuflusses pro Zeiteinheit des Mikrogefäßsystems nach Auslösung der Sprungfunktion erfolgt.

Figur 1

Figur 2

26

$\dfrac{T_D}{T_I}$ ... auf die Herzperiodendauer bezogene (normierte) Dikrotiezeit

a)

$\dfrac{T_G}{T_I}$ ... normierte Gipfelzeit

b)

$\dfrac{S_{TI}}{T_I}$ ...normierte Streuung der Herzperiodendauer $T_I$

c)

Figur 3a-c

Figur 4

Figur 5

Figur 6

Figur 7

Figur 8

Figur 9

Figur 10

34

Figur 11

Fig. 12 a -b

c)

$RPW_2$ opt = 1,618

d)

$J_{rel}$ opt = 1

Fig. 12 c - d

Figur 13a

EP 0 600 298 A1

Figur 13b

EP 0 600 298 A1

zu untersuchende Peripherie
(z.B. Finger)

S $E_1$ $E_2$

S... Sender

$E_1$,$E_2$... Empfänger

Sensor

a

$x_1(t)$ $x_2(t)$

KKF: Ermittlung $\tau_{opt}$

$\tau_{opt}$

$\tau_{opt}$... Verzögerungszeit bei maximaler KKF

$$V_{ström} = \frac{a}{\tau_{opt}}$$

Figur 14

Figur 15

Figur 16

EP 0 600 298 A1

Figur 17 a,b

Figur 18

Figur 19

Figur 20

Figur 21

Figur 22

Figur 23 a, b

EP 0 600 298 A1

EP 0 600 298 A1

Figur 24 a, b

EP 0 600 298 A1

Wirkungsrichtung

Kapillaren

arterielle

venöse

Makrogefäße
(Arterien)

Makrogefäße
(Venen)

Arteriolen

Venolen

Funktionseinheit "Mikrozirkulation"

Figur 25

50

**a)**

x_{mikro}
GP0

Hochlegen des Beines (negativer hydrosr. Druck)

Absenken in Normallage (positiver hydrost. Druck)

$t_{EF}$

$t_{EFarteriell}$ GP1*

Zeit t

"Biopunkt" GP2
(Gleichgewichtszustand beim Füllen)

Entleerung

$t_{EL\ arteriell}$
GP1

arterieller Zufluß

Füllung

$t_{EL\ venös}$

venöser Abfluß

im Mikrozirkulationssystem gespeicherte qualitative Mengenveränderung pro Zeiteinheit

**b)**

negativer    positiver
hydrostatischer Druckserung

Biopunkt GP2

$t_{EL\ venös}$    5s

GP0

(≈20s)

venöser Abfluß

GP1

$t_{EF}$ ≈130s

(≈12s)    arterieller Zufluß

$t_{EL\ arter.}$

**Figur 26**

Figur 27

Figur 28 a, b

arterieller Zufluß

$P_S, P_D [T_B, T_A]$

$R_i$

Peripherie, $R_a$

Herz (zentrale "Pumpanlage")

Herzperiodendauer $T_H$ [ms], Schlagvolumen

venöser Rückfluß

NIR-Sensor: Ermittlung der Volumenpulsation der Mikrozirkulation $x_{mikro}(t)$

Ultraschalldopplersensor: Messung der Strömungsgeschwindigkeit mit den Kennwerten $T_B, T_A$ (Makrobereich)

Kanal 1

Kanal 2

Programmsystem CMMD

CMMD 2

1. **Makrobereich**

   a) Erfassung $P_S, P_D$ bzw. gleichwertig die Zeitparameter $T_B, T_A$

   b) Vergleich mit Normalwerten (Grenzen)

   c) Ermittlung des relativen peripheren Widerstandes $RPW_1$ und Vergleich mit Normalwert

2. **Mikrobereich in Peripherie**

   a) Bestimmung der Einzel- und Mittelwerte der Volumenpulsation:

   $T_H$ ⎫ Einzelwerte der Volumenpulsparameter,
   $T_G$ ⎬ Mittelwerte,
   $T_D$ ⎬ Vergleich mit Normalwerten,
   $T_{AG}$ ⎭ Histogramm Herzperiodendauer, Mikrogefäßzustand $T_G = f(T_D)$

   b) Ermittlung des peripheren Widerstandes $RPW_2$ und Vergleich mit Normalwert

   c) Graphische Darstellung $RPW_2 = f(Volumenpuls)$

4. **Relativer mittlerer (Blut-)Stromfluß $I_{rel}$**

5. **Aus 1.-4. abgeleiteter Diagnosealgorithmus**

*Figur 29*

**Europäisches Patentamt**

# EUROPÄISCHER RECHERCHENBERICHT

Nummer der Anmeldung

EP 93 11 8494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | EP-A-0 359 972 (A.NATTERMANN & CIE. GMBH) | 1,36 | A61B5/024 |
| A | * Spalte 2, Zeile 40 - Spalte 5, Zeile 34; Abbildungen 1-5 * | 4,7,19, 35 | A61B5/0285 |
| | --- | | |
| Y | DE-C-841 934 (ELEKTROFREQUENZ FRITZ SCHWARZER GMBH) | 1,36 | |
| A | * Seite 2, Zeile 36 - Seite 3, Zeile 36; Abbildungen 1-3 * | 2,18-20, 37 | |
| | --- | | |
| A | EP-A-0 329 489 (VASCULAR SURGICAL FORUM) | 1,22,33, 34 | |
| | * Seite 2, Zeile 34 - Seite 3, Zeile 4 * * Seite 3, Zeile 54 - Seite 4, Zeile 12; Abbildungen 1,2 * | | |
| | --- | | |
| A | DE-A-31 46 063 (R.F.STEPHENS) * Seite 2, Zeile 5 - Seite 3, Zeile 4 * * Seite 5, Zeile 12 - Seite 6, Zeile 14 * * Seite 9, Zeile 27 - Seite 10, Zeile 22; Abbildung 1 * | 1,9 | |
| | ----- | | |

**RECHERCHIERTE SACHGEBIETE (Int.Cl.5)**

A61B

Der vorliegende Recherchenbericht wurde für alle Patentansprüche erstellt

| Recherchenort | Abschlußdatum der Recherche | Prüfer |
|---|---|---|
| BERLIN | 15. Februar 1994 | Weihs, J |